# EUROPEAN PATENT APPLICATION

(11) **EP 1 939 209 A1**
(43) Date of publication of application: **02.07.2008**
(21) Application number: 06796921.2
(22) Date of filing: 29.08.2006
(51) Int. Cl.: C07H 15/12

(54) **PROCESS FOR PRODUCTION OF LIPID A ANALOGUE**

(30) Priority: 31.08.2005 US 712431 P; 01.09.2005 JP 2005253044
(71) Applicant: Eisai R&D Management Co., Ltd., Tokyo 112-8088 (JP)
(72) Inventor: TAGAMI, Katsuya, Tsukuba-shi, Ibaraki 3002635 (JP); SATO, Keizo, Tsukuba-shi, Ibaraki 3002635 (JP); MATSUO, Kimihiro, Kamisu-shi, Ibaraki 3140255 (JP); ABE, Taichi, Kamisu-shi, Ibaraki 3140255 (JP); HAGA, Toyokazu, Kamisu-shi, Ibaraki 3140255 (JP)
(74) Representative: HOFFMANN EITLE
(86) International application number: PCT/JP2006/316941
(87) International publication number: WO 2007/026675

(57) **Abstract**

Discloses is a process for producing α-D-glucopyranose, 3-O-decyl-2-deoxy-6-O-[2-deoxy-3-O-[(3R)-3-methoxydecyl]-6-O-methyl-2-[(11Z)-1-oxo-11-octadecenyl]amino]-4-O-phosphono-β-D-glucopyranosyl]-2-[(1,3-dioxotetradecyl)amino]- or 1-(dihydrogen phosphate) tetrasodium salt which is useful as an active ingredient of a pharmaceutical or an intermediate for the synthesis thereof, which is environment-friendly and excellent in safety, operationality and reproducibility. A process for producing a compound represented by the formula (I) comprising the steps of reacting a compound represented by the formula (VIII) with a palladium catalyst in the presence of a nucleopholic agent and treating the product with a sodium source.

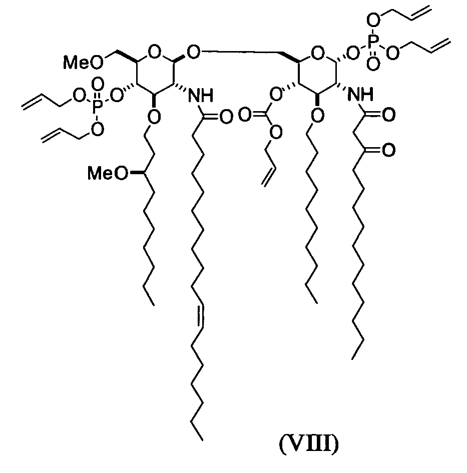

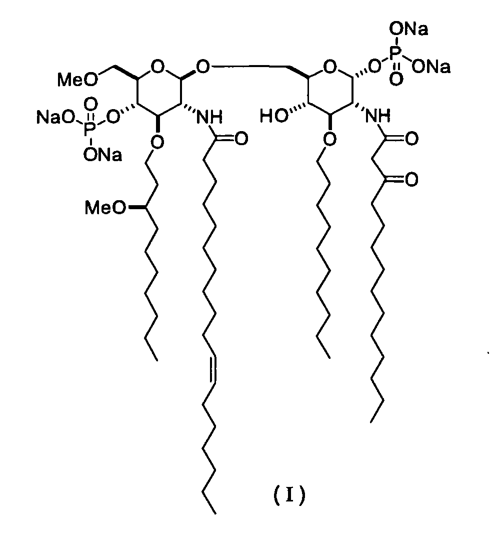

## Description

### Field of the Invention

The present invention relates to a method of preparing a lipid A analog E5564 (known also under the name of B1287, Eritoran) represented by following formula (I), which is useful as medicine.

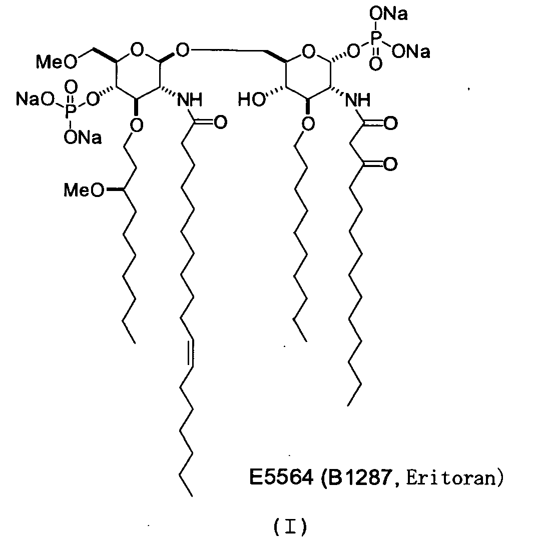

### Background of the Invention

E5564 represented by the formula (I) (B1287, known also under the name of Eritoran) is known to have an excellent effect on prevention or treatment of Gram negative bacteremia, in particular endotoxin shock, manifesting high fatality rate caused by endotoxin or lipopolysaccharide (LPS) components present in Gram negative outer membrane. An excellent anti-endotoxin action of E5564 is confirmed also in a human (Non-Patent Document 1), and E5564 is also known to have an antagonistic action on TLR4 (toll-like receptor 4) which is one of receptors recognizing a fungus body component of a bacterium (Patent Document 1 , Non-Patent Document 2). It is reported that E5564 is particularly useful, based on these actions, as a preventive or therapeutic agent for sepsis, endotoxemia, prognosis of coronary-artery bypass graft surgeries (CABG) and the like (see, e.g., Patent Documents 2, 3 and 4).

Patent Document 2 describes a free form of E5564 and Patent Document 3 describes E5564 (B1287) represented by the formula (I). Further, Patent Documents 5, 6 and 7 disclose a method for preparing E5564.

According to the synthesis method disclosed in Patent Documents 5 , 6 and 7 , E5564 is obtained by bonding two saccharides followed by introducing two acyl-type side chains; however, conversions of functional groups in order to introduce the side chains require more steps, and dichloromethane requires to be used in many steps. Patent Documents 6 and 7 disclose also another synthesis method in which one acyl-type side chain is preintroduced followed by bonding two saccharides; however, introduction of the remaining second acyl-type side chain gives low yield, and use of dichloromethane is also not avoided. Further, Patent Document 3 describes a method in which two acyl-type side chains are preintroduced followed by bonding two saccharides to give a lipid A analog represented by the formula (I). For example, in a step 3 of Example 5 of Patent Document 3 (p. 123 to 124), a compound represented by the formula (VIII) of the present invention is described. However, according to the method described in the step 3, it is necessary, for obtaining a compound represented by the formula (VIII), that using dichloromethane as a solvent, a phosphite group is introduced in the presence of explosive tetrazole followed by adding expensive m-chloroperbenzoic acid as an oxidizing agent at a reaction temperature of -78°C, and then the product is purified by column chromatography. Further, a step 4 (p. 124-125) discloses a method for preparing a compound represented by the formula (II) of the present invention and its tetra sodium salt (B1287) ; however, according to the preparation method, it is necessary to transfer tetrakis(triphenylphosphine) palladium to a reaction can using a nitrogen-filled glove bag. As a synthesis example of saccharides constituting a β-D-glucopyranosyl moiety of a lipid A analog of the present invention, for example, a step 3 in Example 1 (p. 100-101) discloses a method for preparing a compound represented by the formula (III) from the formula (X) according to the present invention; however, its yield is extremely low.
[Patent Document 1] WO2004/071465.
[Patent Document 2] WO96/39411.
[Patent Document 3] WO2004/074303.
[Patent Document 4] US20050153929.
[Patent Document 5] US5750664.
[Patent Document 6] US5935938.
[Patent Document 7] US6417172.
[Non-Patent Document 1] Lynn et al., J. Pharmacol. Exp. Ther. 308 (1) : 175-181, 2004.
[Non-Patent Document 2] Mullarkey et al., J. Pharmacol. Exp. Ther. 304(3): 1093-1102, 2003).

### Disclosure of the Invention

### Problems to be solved by the Invention

Although E5564 shows an excellent action as a preventive or therapeutic agent for sepsis, endotoxemia, prognosis of coronary-artery bypass graft surgeries (CABG) and the like, the known preparation method has problems in the number of steps, raw material initial cost, and safety, operability and reproducibility in the preparation process, and the like, from the standpoint of commercial production of a drug substance. According to the known preparation method, for example, use of dichloromethane as a reaction solvent is necessary in a process of synthesizing E5564; however, according to UN Hazard Class, the dichloromethane is classified into 6.1 [Toxic substances] because of its influence on a human body, and according to International Conference on Harmonization of Technical Requirements for Registration of Pharmaceuticals for Human use (ICH), quality guideline Q3C [guideline regarding impurities: residual solvent], classified into Class 2 [solvents to be limited]. In Japan, the upper limit thereof is set as an environmental standard with regard to air pollution and water contamination.

The preparation method described in Patent Document 3, in which two acyl-type side chains are preintroduced followed by bonding two saccharides, is excellent in decrease in the total number of steps, in particular, in improvement of processes after bonding of saccharides, but shows problems in large amount use of a toxic reagent, use of an explosive reagent, and operability, reproducibility and the like in the preparation process, and additionally, use of dichloromethane is also not avoided.

Thus, there are needs for a method for preparing E5564, which is environmentally friendly, and excellent in safety, operability and reproducibility.

### Means for Solving the Problems

The present inventors have intensively studied, and resultantly found a novel method for preparing E5564 represented by the formula (I) and a novel method which is environmentally-friendly and excellent in safety, operability and reproducibility for preparing its synthesis intermediate, completing the following present invention.
<1> A method for preparing a compound represented by following formula (I), comprising the steps of:
   reacting a compound represented by following formula (VIII) with a palladium catalyst in the presence of a nucleophilic reagent; and
   then treating the resultant with a sodium source to obtain the compound represented by the formula (I). 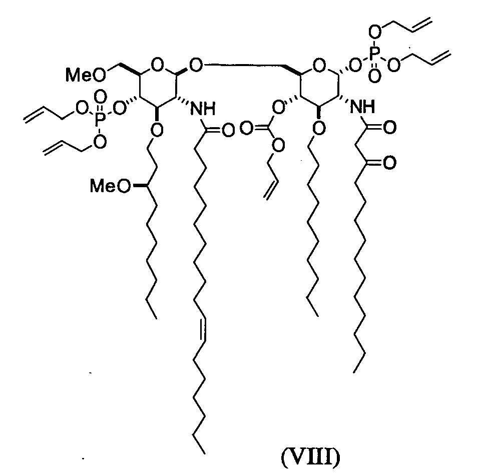 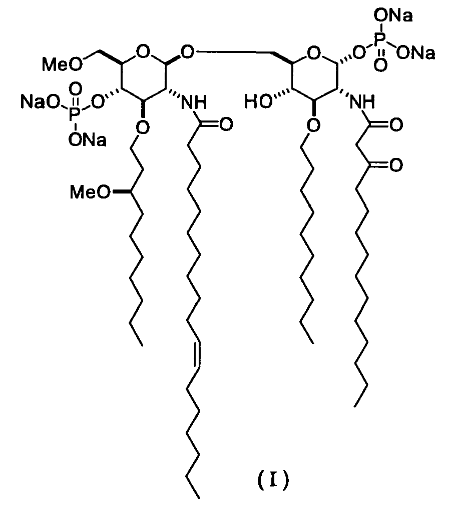
<2> A method for preparing a compound represented by following formula (I), comprising the steps of:
   reacting a compound represented by following formula (VII), diallyl N, N-diisopropylphosphoramidate and an oxidizing agent in this order, in a first aromatic hydrocarbon solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 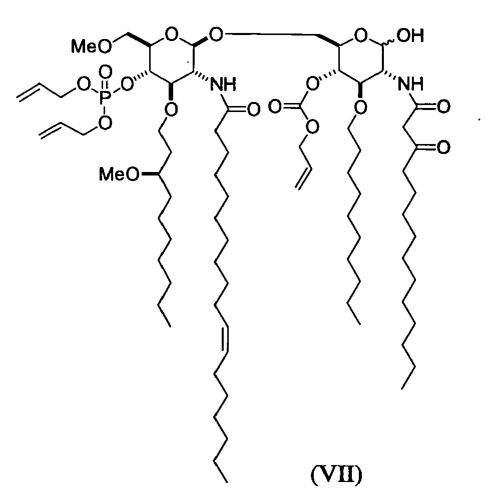 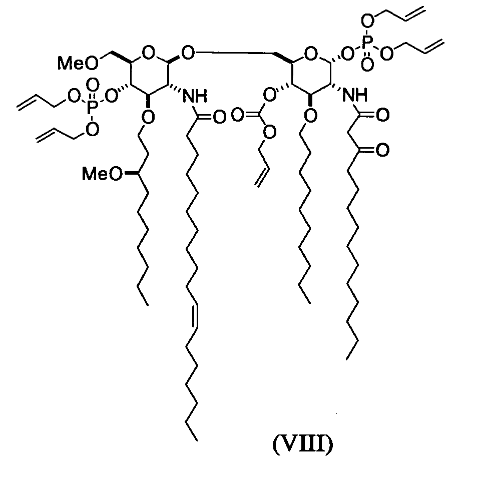 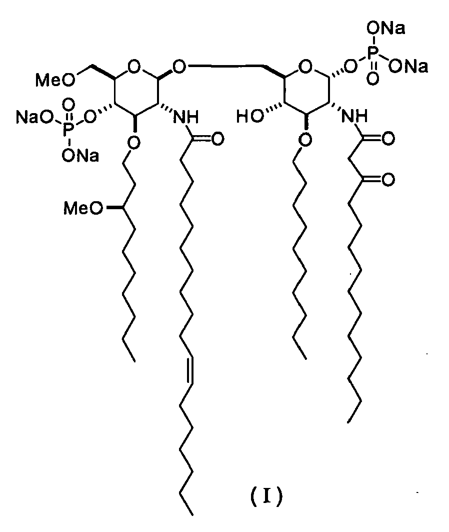
<3> A method for preparing a compound represented by following formula (I), comprising the steps of:
   selectively deprotecting a 1-propenyl group of a compound represented by following formula (VI), to obtain a compound represented by following formula (VII);
   reacting the compound represented by the formula (VII), diallyl N,N-diisopropylphosphoramidate and an oxidizing agent in this order, in a first aromatic hydrocarbon solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 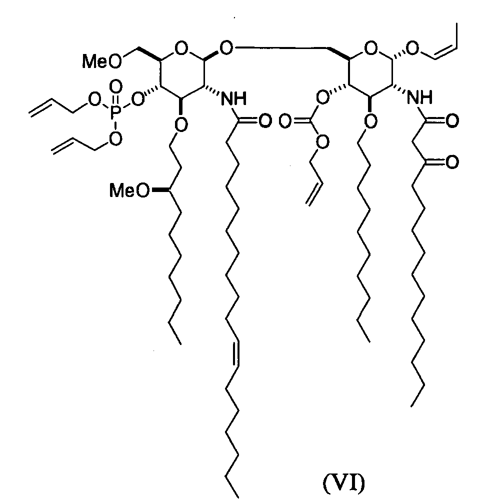 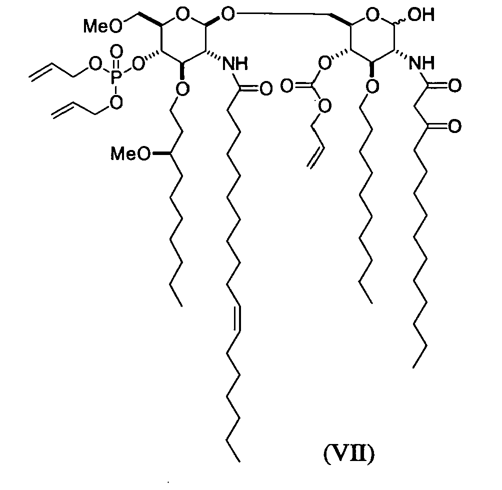 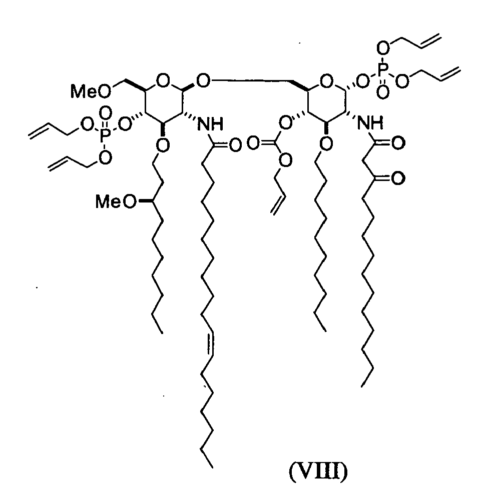 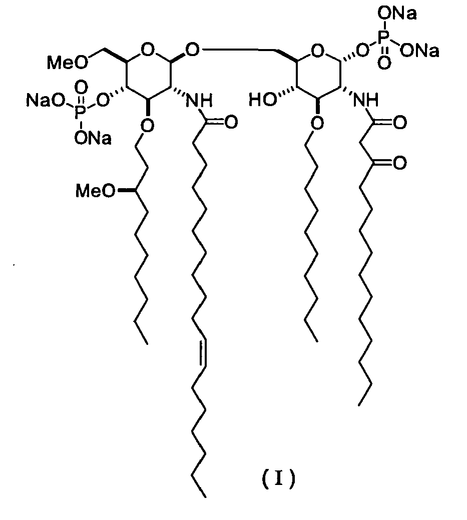
<4> A method for preparing a compound represented by following formula (I), comprising the steps of:
   reacting a compound represented by following formula (IV) and a compound represented by following formula (V) in a first solvent comprising a hydrocarbon solvent and/or a second aromatic hydrocarbon solvent, in the presence of organic sulfonic acid, to obtain a compound represented by following formula (VI);
   selectively deprotecting a 1-propenyl group of the compound represented by the formula (VI), to obtain a compound represented by following formula (VII);
   reacting the compound represented by the formula (VII), diallyl N,N-diisopropylphosphoramidate and an oxidizing agent in this order, in a first aromatic hydrocarbon solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 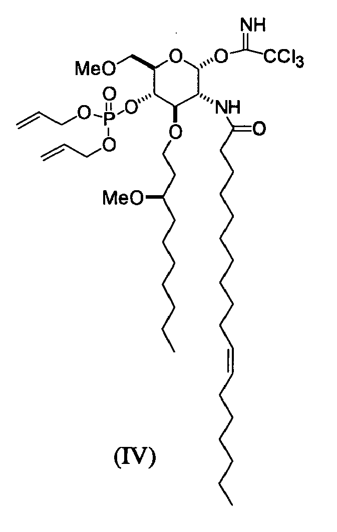 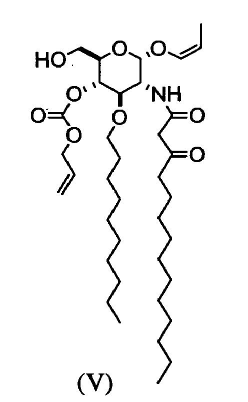 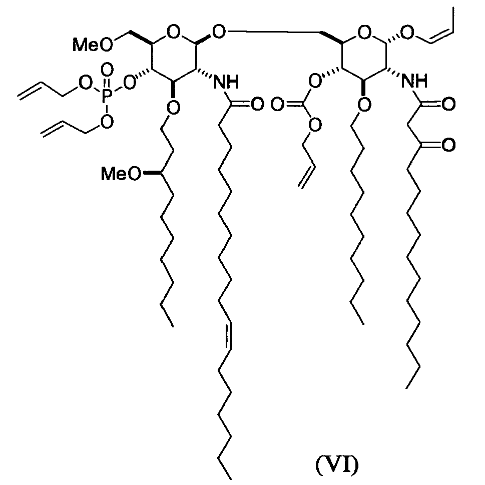 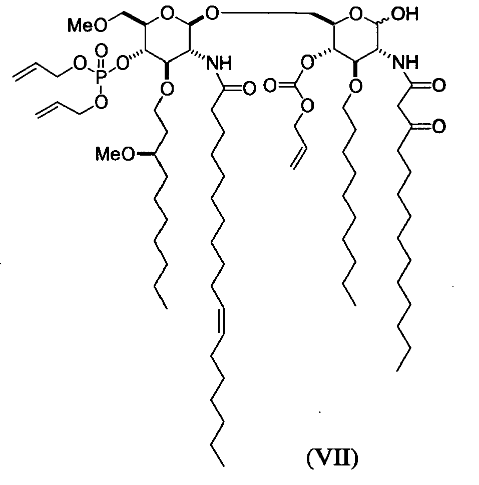 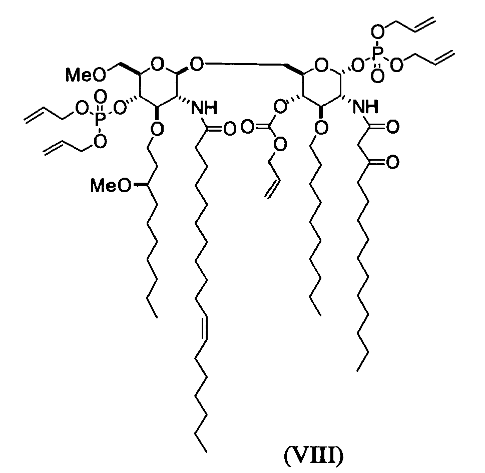 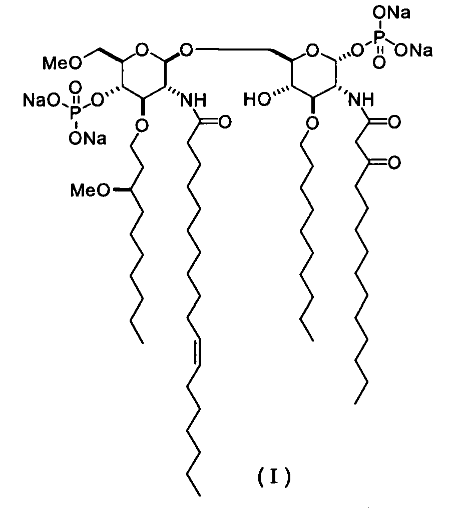
<5> In any one of the above items <1> to <4>, the first aromatic hydrocarbon solvent may be a toluene solvent.
<6> In the above item <4> or <5>, the organic sulfonic acid may be methanesulfonic acid or ethanesulfonic acid.
<7> In any one of the above items <4> to <6>, the first solvent may be a toluene-heptane mixed solvent.
<8> In any one of the above items <4> to <7>, the compound represented by the formula (IV) may be obtained by reacting a compound represented by following formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 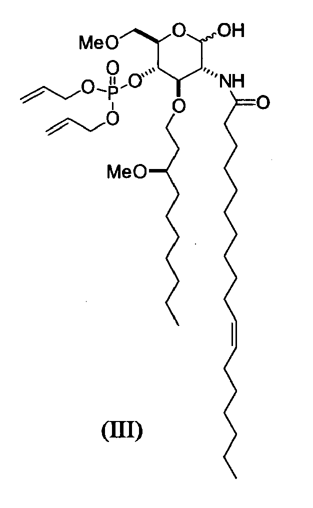
<9> In any one of the above items <4> to <8>, the compound represented by the formula (IV) may be obtained by selectively deprotecting a 1-propenyl group of a compound represented by following formula (X), to obtain a compound represented by following formula (III), and
   then reacting the compound represented by the formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 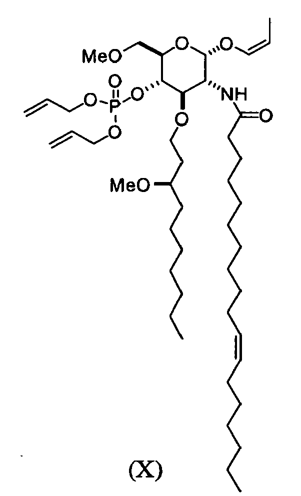 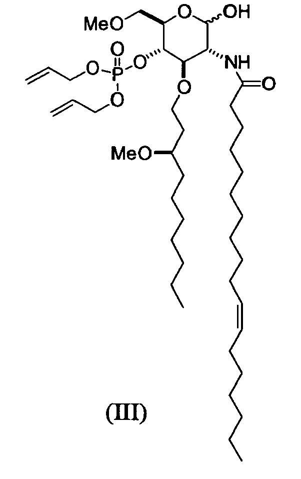
<10> In any one of the above items <4> to <9>, the compound represented by the formula (IV) may be obtained by reacting a compound represented by following formula (IX) with diallyl N,N-diisopropylphosphoramidate and an oxidizing agent in this order, in the presence of pyridine-trifluoroacetic acid, to obtain a compound represented by following formula (X);
   selectively deprotecting a 1-propenyl group of the compound represented by the formula (X), to obtain a compound represented by following formula (III), and
   then reacting the compound represented by the formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 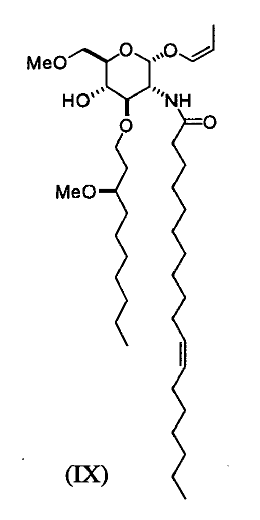 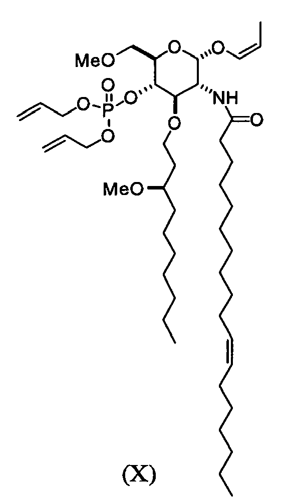 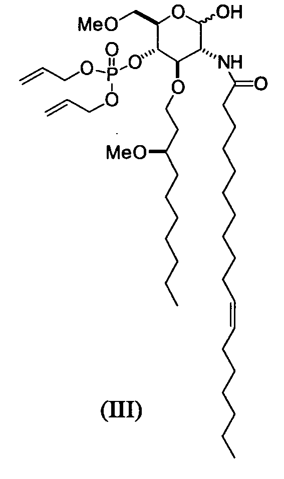
<11> In any one of the above items <8> to <10>, the acetate ester solvent may be methyl acetate.
<12> In any one of the above items <8> to <11>, an amount of water in the mixed solvent may range 1 to 10 percent (vol/vol ratio).
<13> In any one of the above items <1> to <12>, the nucleophilic reagent may be cyclic organic acid esters or cyclic ketones.
<14> In any one of the above items <1> to <13>, the nucleophilic reagent may be Meldrum's acid or Dimedone.
<15> In any one of the above items <1> to <14>, the palladium catalyst may be tetrakis(triphenylphosphine) palladium.
<16> In the above item <15>, the tetrakis (triphenylphosphine) palladium may be prepared in situ from palladium acetate and triphenylphosphine.
<A1> A method for preparing a compound represented by following formula (I), comprising the steps of:
   reacting a compound represented by following formula (VIII) with a palladium catalyst in the presence of a nucleophilic reagent; and
   then treating the resultant with a sodium source to obtain the compound represented by the formula (I). 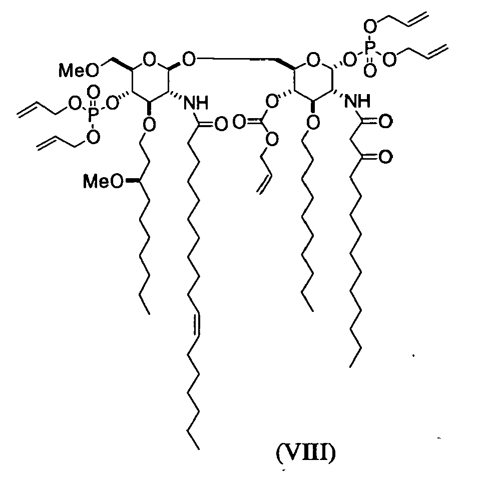 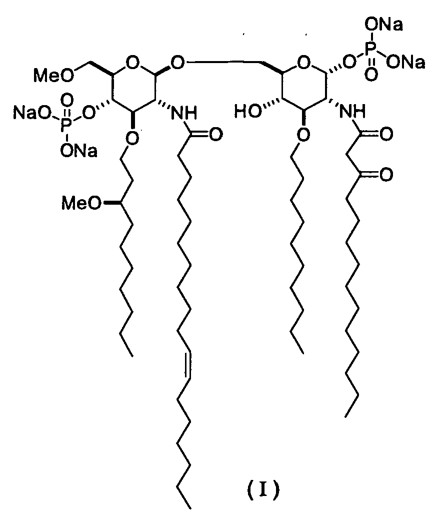
<A2> A method for preparing a compound represented by following formula (I), comprising the steps of:
   reacting a compound represented by following formula (VII), diallyl N,N-diisopropylphosphoramidate and an oxidizing agent in this order, in a toluene solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 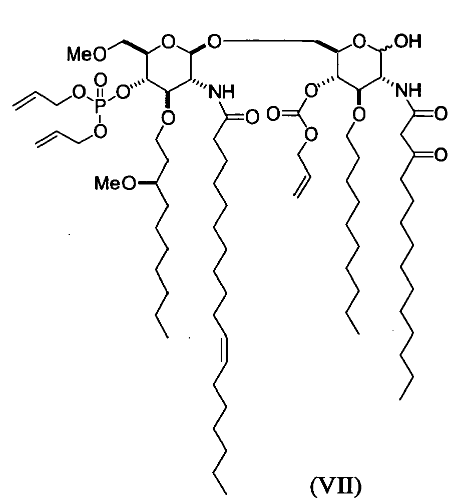 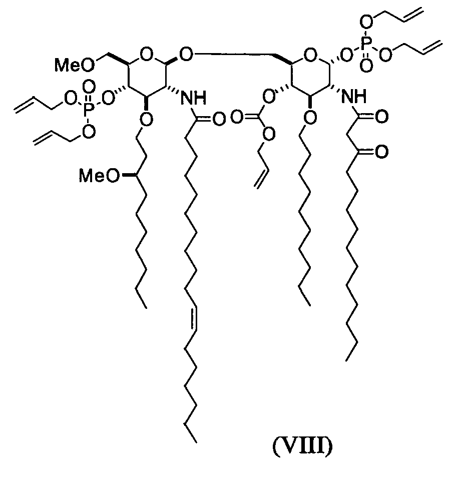 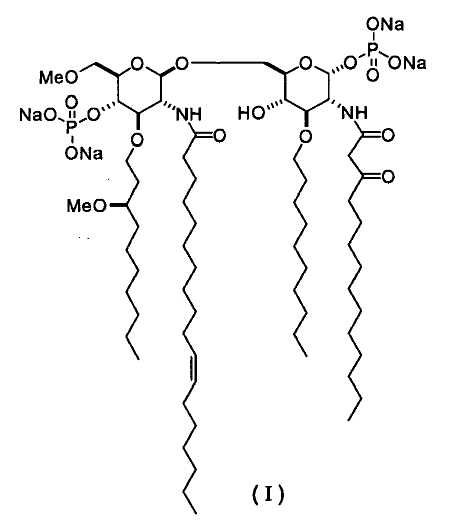
<A3> A method for preparing a compound represented by following formula (I), comprising the steps of:
   selectively deprotecting a 1-propenyl group of a compound represented by following formula (VI), to obtain a compound represented by following formula (VII);
   reacting the compound represented by the formula (VII), diallyl N,N-diisopropylphosphoramidate and an oxidizing agent in this order, in a toluene solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 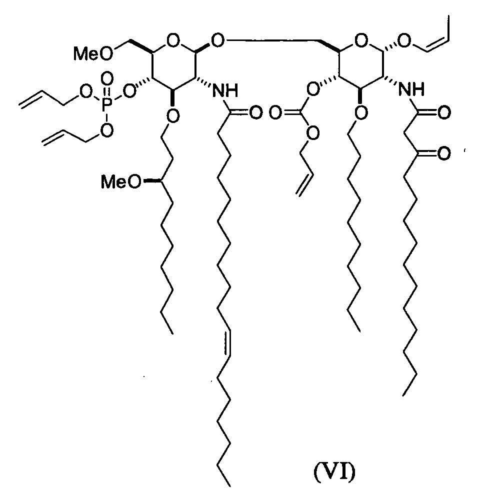 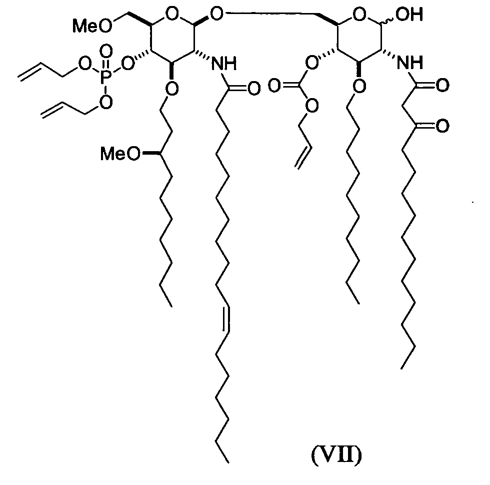 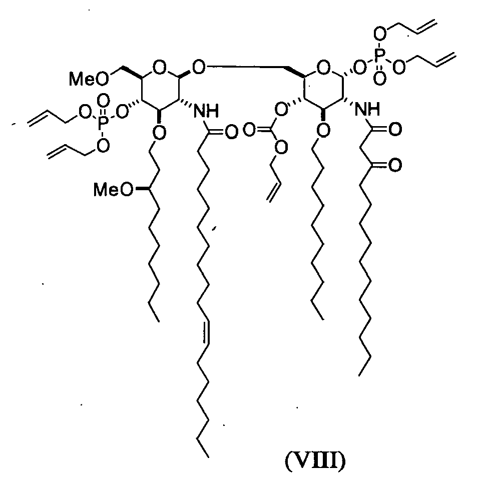 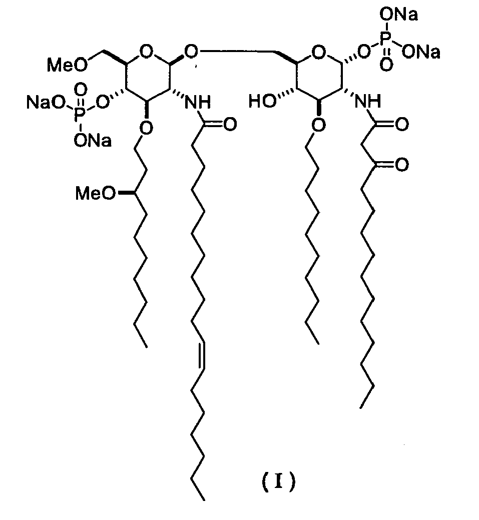
<A4> A method for preparing a compound represented by following formula (I), comprising the steps of:
   reacting a compound represented by following formula (IV) and a compound represented by following formula (V) in a toluene-heptane mixed solvent, in the presence of methanesulfonic acid, to obtain a compound represented by following formula (VI);
   selectively deprotecting a 1-propenyl group of the compound represented by the formula (VI), to obtain a compound represented by following formula (VII);
   reacting the compound represented by the formula (VII), diallyl N,N-diisopropylphosphoramidate and an oxidizing agent in this order, in a toluene solvent in the presence of pyridine-trifluoroacetic acid, to obtain a compound represented by following formula (VIII);
   reacting the compound represented by the formula (VIII) with a palladium catalyst in the presence of a nucleophilic reagent; and
   then treating the resultant with a sodium source to obtain the compound represented by the formula (I). 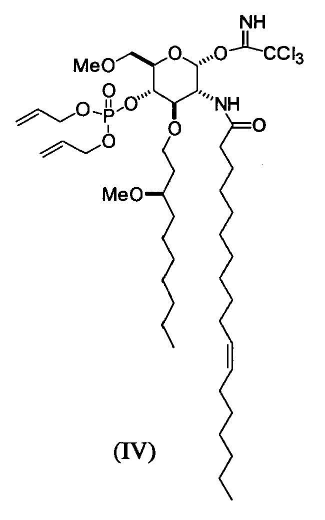 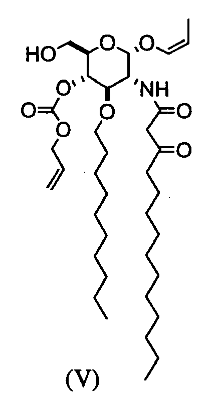 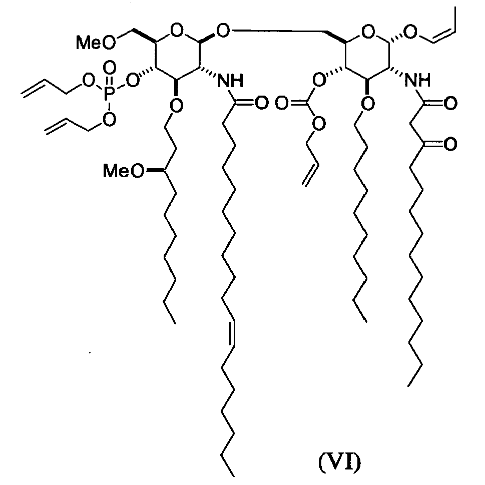 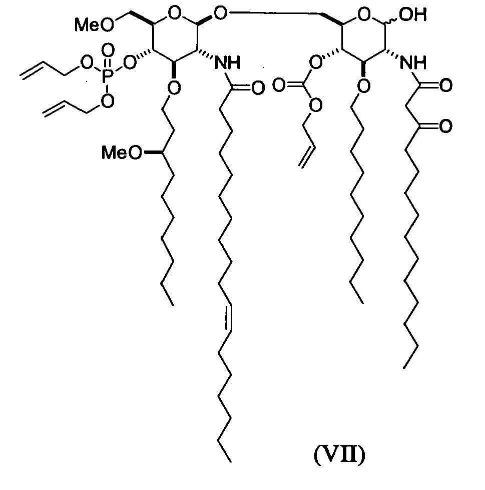 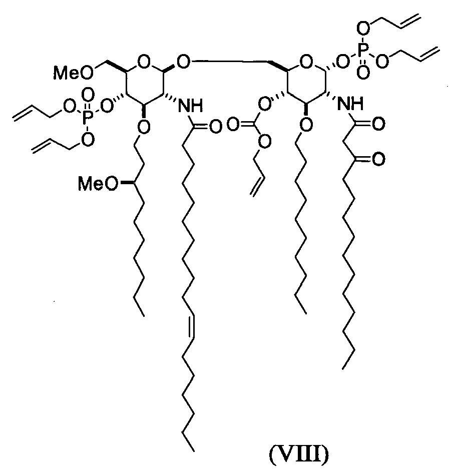 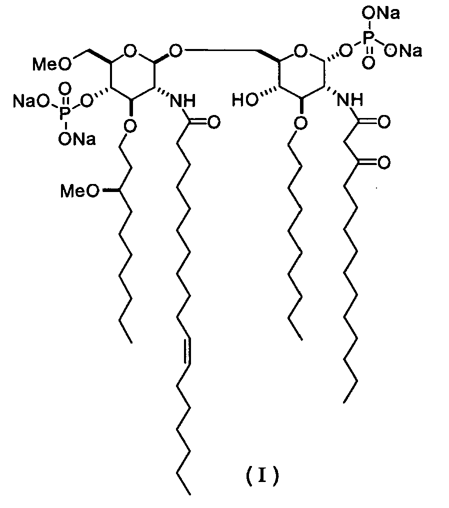
<A5> In the above item <A4>, the compound represented by the formula (IV) may be obtained by reacting a compound represented by following formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 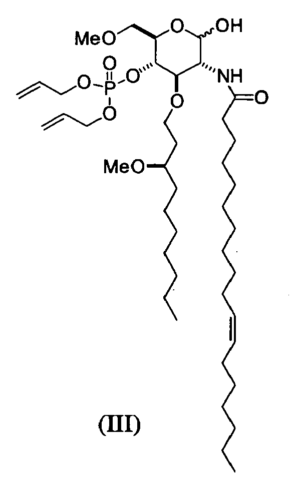
<A6> In the above item <A4>, the compound represented by the formula (IV) may be obtained by selectively deprotecting a 1-propenyl group of a compound represented by following formula (X), to obtain a compound represented by following formula (III), and
   then reacting the compound represented by the formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 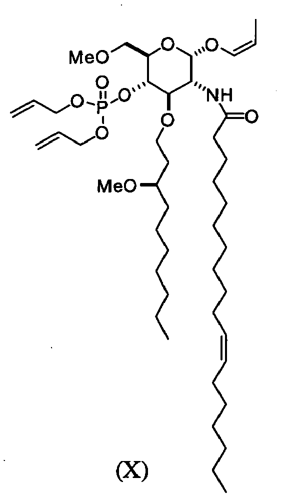 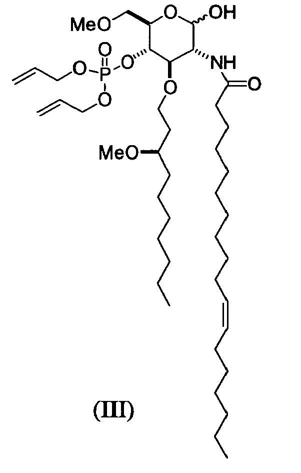
<A7> In the above item <A4>, the compound represented by the formula (IV) may be obtained by reacting a compound represented by following formula (IX) with diallyl N,N-diisopropylphosphoramidate and an oxidizing agent in this order, in the presence of pyridine-trifluoroacetic acid, to obtain a compound represented by following formula (X);
   selectively deprotecting a 1-propenyl group of the compound represented by the formula (X), to obtain a compound represented by following formula (III), and
   then reacting the compound represented by the formula (III) and trichloroacetonitrile in a mixed solvent of an acetate ester solvent and water, in the presence of potassium carbonate, in which an amount of trichloroacetonitrile may range 1 to 10 equivalents based on 1 equivalent of the compound represented by the formula (III). 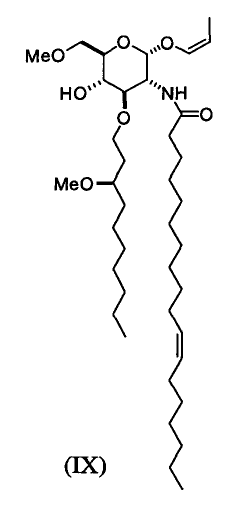 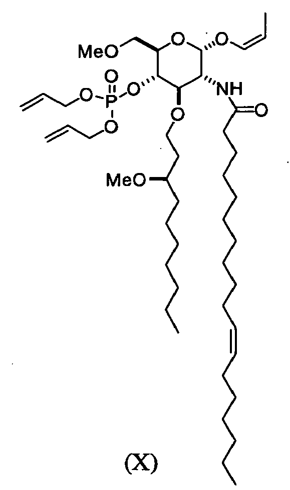 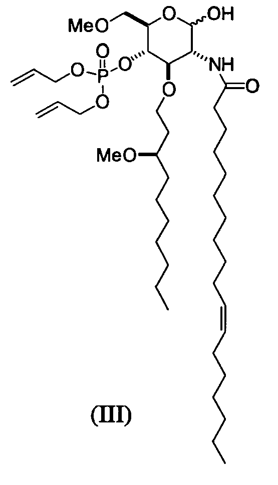
<A8> In any one of the above items <A5> to <A7>, the acetate ester solvent may be methyl acetate.
<A9> In any one of the above items <A5> to <A8>, an amount of water in the mixed solvent may range 1 to 10 percent (vol/vol ratio).
<A10> In any one of the above items <A1> to <A9>, the nucleophilic reagent may be cyclic organic acid esters or cyclic ketones.
<A11> In any one of the above items <A1> to <A10>, the nucleophilic reagent may be Meldrum's acid or Dimedone.
<A12> In any one of the above items <A1> to <A11>, the palladium catalyst may be tetrakis(triphenylphosphine) palladium.
<A13> In the above item <A12>, the tetrakis (triphenylphosphine) palladium may be prepared in situ from palladium acetate and triphenylphosphine.

### Effect of the Invention

The present invention can produce, as a drug substance, a compound (I) (E5564) which is particularly useful as a preventive or therapeutic agent for sepsis, endotoxemia and prognosis of coronary-artery bypass graft surgeries (CABG) since it antagonizes lipid A playing an important role in Gram negative bacteremia, in particularly endotoxin shock, manifesting high fatality rate caused by lipopolysaccharide (LPS) components or endotoxin present in Gram negative outer membrane, shows an excellent anti-endotoxin action, and shows an antagonistic action on TLR4 (toll-like receptor 4) which is one of receptors recognizing a fungus body component of a bacterium.

### Best Modes for Carrying out the Invention

The following abbreviations may be used herein.
DDP: diallyl N,N-diisopropylphosphoramidate;
Py: pyridine; and
TFA: trifluoroacetic acid.

Hereinafter, the method for preparing a compound of the formula (I) according to the present invention will be described in detail.

The compound of the formula (I) can be prepared by the following preparation method.

### Preparation method

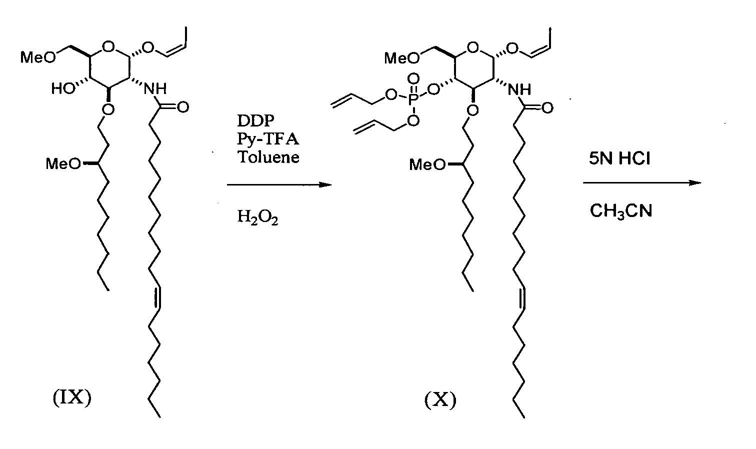

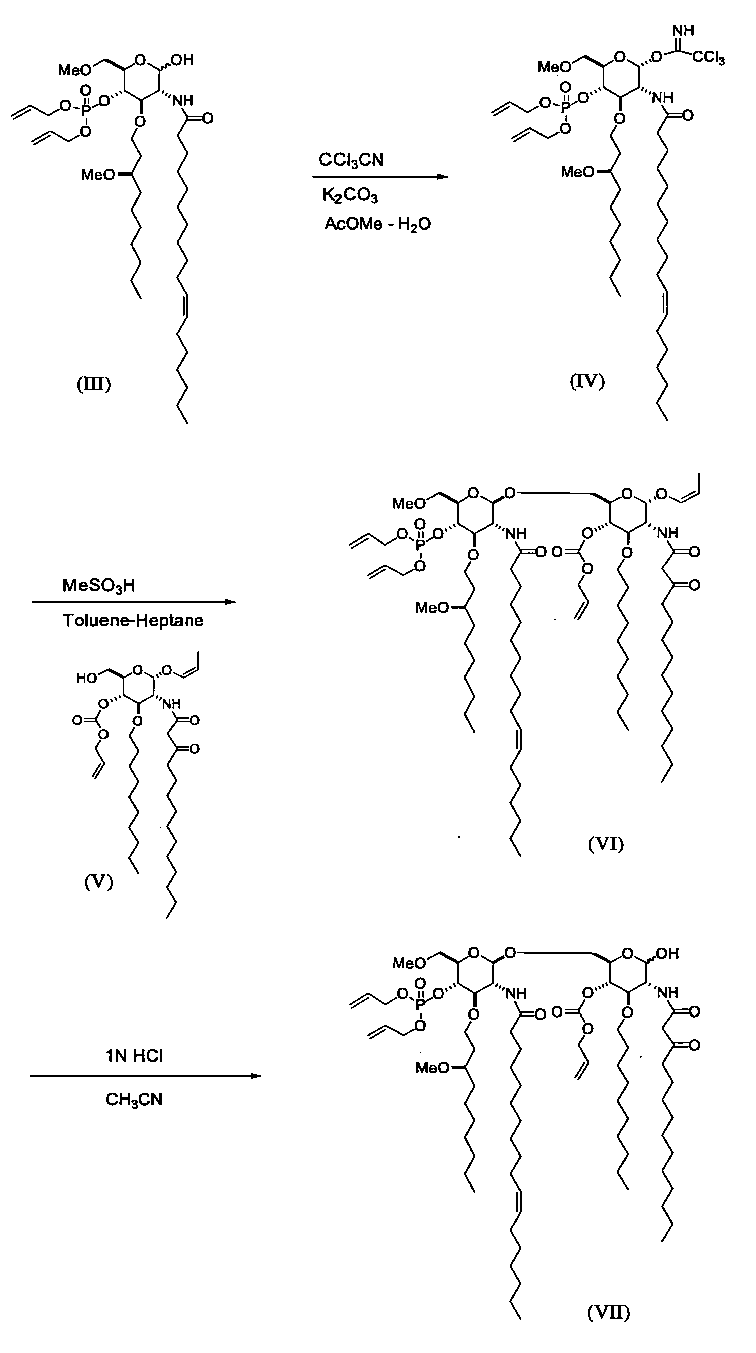

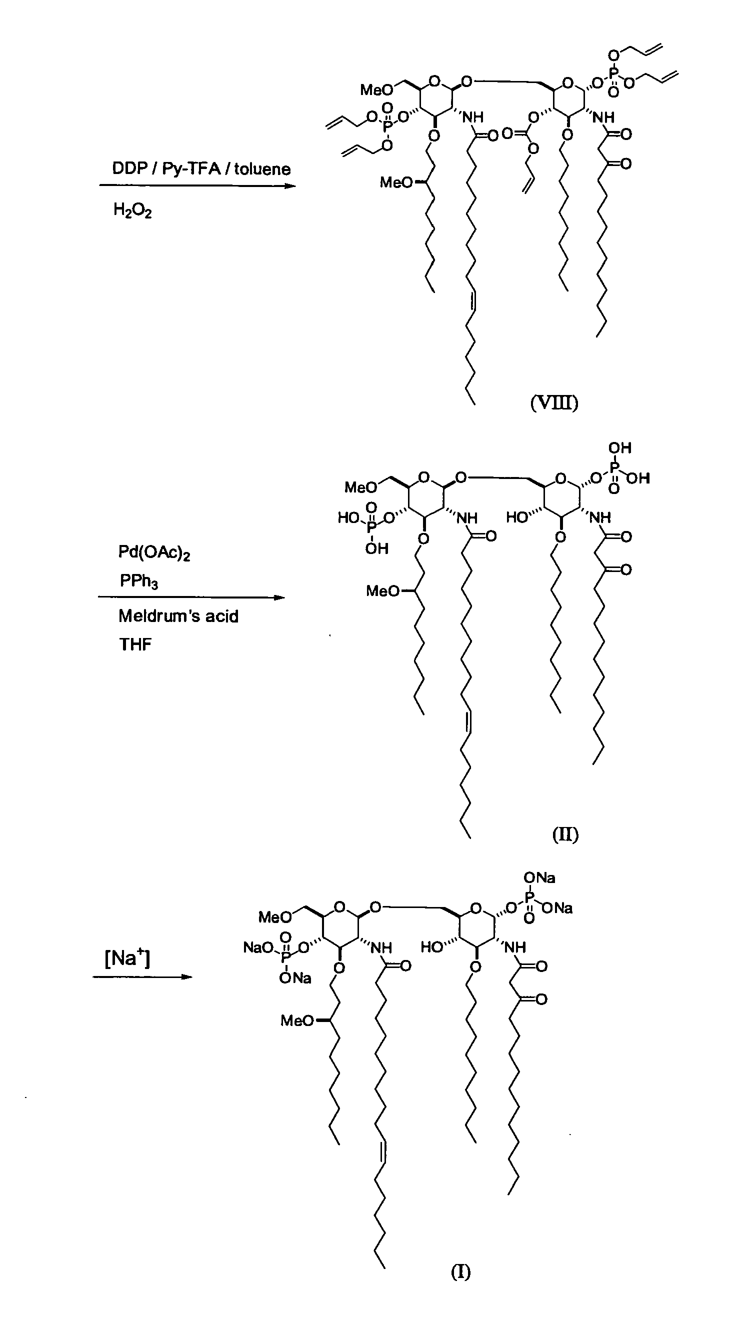

The first step of the present preparation method is a process in which a phosphite group is introduced into a compound of the formula (IX) followed by an oxidation reaction, to obtain a compound of the formula (X). The solvent to be used in this step is not particularly limited. It is desirable that the solvent may be one of inert solvents not reacting easily with a raw material. Examples thereof may include ethers such as tetrahydrofuran, diethyl ether, diisopropyl ether, dioxane, dimethoxyethane and the like; halogenated hydrocarbons such as chloroform, carbon tetrachloride, 1,2-dichloroethane and the like; hydrocarbons such as hexane, heptane and the like; aromatic hydrocarbons such as benzene, toluene and the like; acetate esters such as ethyl acetate, methyl acetate and the like; amides such as N,N-dimethylformamide, N-methyl-2-piperidone, hexamethylphosphorylamide and the like; sulfoxides such as dimethyl sulfoxide and the like; and mixed solvents thereof; and the like. Among them, aromatic hydrocarbon solvents are preferable, and particularly, for example, toluene is more preferable.

The presence of pyridine and trifluoroacetic acid allowed the reaction of this step to be carried out under mild conditions . Pyridine and trifluoroacetic acid to be used in this step can be used in equal amounts or excess amounts based on the amount of a compound of the formula (IX) . In view of the smooth reaction and purification treatment and the like, the amounts used thereof may be preferably 1. 0 to 3. 0 equivalents and 1. 0 to 3. 0 equivalents, in particular more preferably 1.0 to 2.0 equivalents and 1.0 to 2.0 equivalents, respectively.

This step consists of two processes: a step of introducing a phosphite group and an oxidation step. Diallyl N,N-diisopropylphosphoramidate used in the step of introducing a phosphite group can be used in equivalent or excess amount based on the amount of a compound of the formula (IX), and the amount may be preferably 1.0 to 2.0 equivalents. The reaction time of the step of introducing a phosphite group may be 0.5 to 4 hours, preferably 1 to 2 hours. The reaction temperature may be -78°C to room temperature, preferably -40 to 0°C. The oxidizing agent to be used in the oxidation step may include hydrogen peroxide, m-chloroperbenzoic acid, oxone and the like, most preferably hydrogen peroxide. Hydrogen peroxide can be used in equal amount or excess amount based on the amount of a compound of the formula (IX), and 1.0 to 3.0 equivalents are preferable. The reaction time of the oxidation step may be 0.5 to 6 hours, preferably 1 to 4 hours. The reaction temperature may be preferably -50 to 0°C.

The second step of the present preparation method is a process in which a 1-propenyl group is selectively deprotected from a compound of the formula (X) through acid hydrolysis to prepare a compound of the formula (III) . The solvent to be used in this step is not particularly limited. It is desirable that the solvent may be one of inert solvents not reacting easily with a raw material. Examples thereof may include alcohols such as methanol, ethanol, isopropanol, tert-butanol and the like; ethers such as tetrahydrofuran, diethyl ether, diisopropyl ether, dioxane, dimethoxyethane, diethoxyethane, diglyme and the like; halogenated hydrocarbons such as chloroform, carbon tetrachloride, 1,2-dichloroethane and the like; hydrocarbons such as hexane, haptane and the like; aromatic hydrocarbons such as benzene, toluene and the like; nitriles such as acetonitrile and the like; amides such as N,N-dimethylformamide, N,N-dimethylacetamide, N-methyl-2-piperidone, hexamethylphosphorylamide and the like; and sulfoxides such as dimethyl sulfoxide and the like. Among them, nitriles such as acetonitrile and the like are preferable.

The acid to be used in this step may include general organic acids and inorganic acids. Examples of the organic acid may include mono-carboxylic acids such as acetic acid, trifluoroacetic acid, propionic acid, benzoic acid and the like; di-carboxylic acids such as oxalic acid and the like; and organic sulfonic acid such as methanesulfonic acid, tosylic acid, trifluoromethanesulfonic acid and the like. Examples of the inorganic acid may include phosphoric acid, hydrochloric acid, sulfuric acid and nitric acid. Inorganic acids such as hydrochloric acid, sulfuric acid and the like are preferable.

The acid to be used in this step can be used in catalytic amount to excess amount based on the amount of a compound of the formula (X) . In view of the smooth reaction and purification treatment and the like, the amount used may be preferably 0.01 to 1.5 equivalents, more preferably 0.1 to 1.0 equivalents.

The reaction time may be 0.5 to 12 hours, preferably 1 to 6 hours. The reaction temperature may be 0°C to reflux temperature, preferably 10 to 60°C.

The resultant compound of the formula (III) is treated under best conditions to give a crystal, obtaining an effect of improving purity, and the like.

The third step of the present preparation method is a process in which a trichloroethane imidate group is introduced as a releasing group into a compound of the formula (III) in the presence of a base, to produce a compound of the formula (IV). Trichloroacetonitrile to be used in this step can be used in equal amount to excess amount based on the amount of a compound of the formula (III). In view of the smooth reaction and purification treatment and the like, the amount used may be preferably 1.0 to 10.0 equivalents, more preferably 2.0 to 5.0 equivalents.

The solvent to be used in this step may be ethers such as tetrahydrofuran, diethyl ether, diisopropyl ether, dioxane, dimethoxyethane and the like; halogenated hydrocarbons such as chloroform, carbon tetrachloride, 1,2-dichloroethane and the like; acetate esters such as methyl acetate, ethyl acetate and the like; water; mixed solvents thereof; and the like. Among them, mixed solvents of water and acetate esters such as methyl acetate, ethyl acetate and the like are suitable, since the reaction can be carried out with good reproducibility with such solvents.

Regarding the mixing ratio of acetate esters to water to be used as the solvent, the proportion of water may be 1 to 10% (vol/vol ratio), suitably 2 to 5%.

The base to be used in this step may be carbonates such as sodium carbonate, potassium carbonate, cesium carbonate and the like; hydrogencarbonates such as sodium hydrogencarbonate and the like; and alkali metal alkoxides such as sodiummethoxide, potassium tert-butoxide and the like. Among them, carbonates such as potassium carbonate and the like are preferable.

The base to be used in this step can be used in equal amount or excess amount based on the amount of compound of the formula (III). In view of the smooth reaction and purification treatment and the like, the amount used thereof may be preferably 0.5 to 3. 0 equivalents, more preferably 1.0 to 1. 3 equivalents.

The reaction time may be 0.5 to 24 hours, preferably 1 to 5 hours. The reaction temperature may be preferably -20°C to room temperature, more preferably -5 to 10°C.

The fourth step of the present preparation method is a process in which a compound of the formula (IV) and a compound of the formula (V) are glycosyl-bonded to prepare a compound of the formula (VI). The glycosylation reaction can be carried out in the presence of an acid catalyst. The acid catalyst to be used in this step may include organic acids and Lewis acid, preferably organic sulfonic acids such as methanesulfonic acid, ethanesulfonic acid, camphorsulfonic acid, p-toluenesulfonic acid and the like, and more preferably methanesulfonic acid and ethanesulfonic acid.

The solvent to be used in this step may be desirably one of inert solvents not reacting easily with a raw material. Examples thereof may include ethers such as tetrahydrofuran, diethyl ether, diisopropyl ether, dioxane, dimethoxyethane and the like; halogenated hydrocarbons such as chloroform, carbon tetrachloride, 1,2-dichloroethane and the like; hydrocarbons such as hexane, heptane and the like; aromatic hydrocarbons such as benzene, toluene and the like; and nitriles such as acetonitrile and the like; and mixtures thereof. Among them, hydrocarbons such as hexane, heptane and the like; aromatic hydrocarbons such as benzene, toluene and the like; and mixed solvents thereof are preferable, and particularly a mixed solvent of heptane and toluene is preferable. The reaction temperature may be 0°C to reflux temperature, preferably 10 to 30°C. The reaction time can be 1 hour to 7 days, preferably 8 hours to 3 days.

The fifth step of the present preparation method is a process in which a 1-propenyl group is selectively deprotected from a compound of the formula (VI) by acid hydrolysis to prepare a compound of the formula (VII). The solvent to be used in this step is not particularly limited. It is desirable that the solvent may be one of inert solvents not reacting easily with a raw material. Examples thereof may include alcohols such as methanol, ethanol, isopropanol, tert-butanol and the like; ethers such as tetrahydrofuran, diethylether, diisopropyl ether, dioxane, dimethoxyethane, diethoxyethane, diglyme and the like; halogenated hydrocarbons such as chloroform, carbon tetrachloride, 1,2-dichloroethane and the like; hydrocarbons such as hexane, haptane and the like; aromatic hydrocarbons such as benzene, toluene and the like; nitriles such as acetonitrile and the like; amides such as N,N-dimethylformamide, N,N-dimethylacetamide, N-methyl-2-piperidone, hexamethylphosphorylamide and the like; and sulfoxides such as dimethyl sulfoxide and the like. Among them, nitriles such as acetonitrile and the like are preferable.

The acid to be used in this step may include general organic acids and inorganic acids. Examples of the organic acid may include mono-carboxylic acids such as acetic acid, trifluoroacetic acid, propionic acid, benzoic acid and the like; di-carboxylic acids such as oxalic acid and the like; and organic sulfonic acids such as methanesulfonic acid, tosylic acid, trifluoromethanesulfonic acid and the like. Examples of the inorganic acid may include phosphoric acid, hydrochloric acid, sulfuric acid and nitric acid. Inorganic acids such as hydrochloric acid, sulfuric acid and the like are preferable.

The acid to be used in this step can be used in catalytic amount to excess amount based on the amount of a compound of the formula (VI). In view of the smooth reaction and purification treatment and the like, the amount used may be preferably 0.01 to 1.5 equivalents, more preferably 0.1 to 0.5 equivalents.

The reaction time may be 0.5 to 12 hours, preferably 1 to 6 hours. The reaction temperature may be 0°C to reflux temperature, preferably 10 to 60°C.

The reaction and treatment under reduced pressure in this step give effects of enhanced yield, improved operability, reduced by-products and the like.

The sixth step of the present preparation method is a process in which phosphite group is introduced into a compound of the formula (VII) followed by an oxidation reaction, to obtain a compound of the formula (VIII). The solvent to be used in this step is not particularly limited. It is desirable that the solvent may be one of inert solvents not reacting easily with a raw material. Examples thereof may include ethers such as tetrahydrofuran, diethyl ether, diisopropyl ether, dioxane, dimethoxyethane and the like; halogenated hydrocarbons such as chloroform, carbon tetrachloride, 1,2-dichloroethane and the like; hydrocarbons such as hexane, heptane and the like; aromatic hydrocarbons such as benzene, toluene and the like; acetate esters such as ethyl acetate, methyl acetate and the like; amides such as N,N-dimethylformamide, N-methyl-2-piperidone, hexamethylphosphorylamide and the like; sulfoxides such as dimethyl sulfoxide and the like; and mixed solvents thereof; and the like. Among them, aromatic hydrocarbon solvents are preferable, and particularly, for example, toluene is more preferable.

The presence of pyridine and trifluoroacetic acid allowed the reaction of this step to be carried out under mild conditions. Pyridine and trifluoroacetic acid to be used in this step can be used in equal amounts or excess amounts based on the amount of a compound of the formula (VII). In view of the smooth reaction and purification treatment and the like, the amounts used thereof may be preferably 1.0 to 3.0 equivalents and 1. 0 to 3. 0 equivalents, particularly, 1.0 to 2.0 equivalents and 1.0 to 2.0 equivalents, respectively.

This step consists of two processes: a step of introducing a phosphite group and an oxidation step. Diallyl N,N-diisopropylphosphoramidate used in the step of introducing a phosphite group can be used in equivalent or excess amount based on the amount of a compound of the formula (VII), and preferably 1.5 to 3.0 equivalents. The reaction time of the step of introducing a phosphite group may be 0.5 to 24 hours, preferably 0.5 to 4 hours. The reaction temperature may be -78°C to room temperature, preferably -40 to 0°C. The oxidizing agent to be used in the oxidation step may include hydrogen peroxide, m-chloroperbenzoic acid, oxone and the like, and most preferably hydrogen peroxide. The reaction time of the oxidation step may be 0.5 to 6 hours, preferably 1 to 3 hours. The reaction temperature may be preferably -50 to 0°C.

The seventh step of the present preparation method is a process in which 2-propenyl groups of a compound of the formula (VIII) are deprotected to prepare a compound of the formula (II). Removal of the 2-propenyl group can be carried out by methods described in documents, for example, hydrolysis using an acid or base, deallylation reaction using a metal catalyst such as a palladium catalyst, and the like. Among them, the deallylation reaction using a metal catalyst such as, for example, a palladium catalyst and the like is preferable, and use of a 0-valent palladium catalyst such as tetrakis(triphenylphosphine)palladium and the like is more preferable. As the 0-valent palladium catalyst such as tetrakis(triphenylphosphine)palladium and the like, commercially available reagents can be used; however, a method for generating the catalyst in a system is preferable from the standpoint of stability of the reagent. For example, a combination of a di-valent palladium reagent with a ligand such as triphenylphosphine and the like is preferable. The di-valent palladium reagent to be used in this step may include palladium acetate, palladium chloride, bis (triphenylphosphine) palladium (II) chloride and the like. For example, when palladium acetate is used as the di-valent palladium reagent, palladium acetate can be used in catalytic amount based on the amount of a compound of the formula (VIII). In view of the smooth reaction and purification treatment and the like, the amount used thereof may be preferably 0.01 to 0.50 equivalents, more preferably 0.05 to 0.25 equivalents. Triphenylphosphine can be used in an amount of 1.5 to 10 equivalents based on the amount of a compound of the formula (VIII), and the amount used may be more preferably 3.0 to 5.0 equivalents. The nucleophilic reagent to be used in this reaction may be preferably a compound having an active methylene structure in the molecule. Examples thereof may include linear organic acid esters such as ethyl cyanoacetate and the like; cyclic organic acid esters such as Meldrum's acid (Isopropylidene malonate) and the like; and cyclic ketones such as dimedone (5,5-Dimethyl-1,3-cyclohexanedione) and the like. Among them, preferable are cyclic organic acid esters such as Meldrum's acid and the like and cyclic ketones such as dimedone and the like from the standpoint of reduced by-products.

The nucleophilic reagent to be used in this step can be used in equal amount or excess amount, preferably 10 to 100 equivalents, more preferably about 20 to 30 equivalents based on the amount of palladium acetate. The reaction time may be 1 to 12 hours, preferably 2 to 6 hours. The reaction temperature may be 10°C to 50°C, preferably 20°C to 40°C.

The solvent to be used in this step is not particularly limited. It is desirable that the solvent may be one of inert solvents not reacting easily with a raw material. Examples thereof may include ethers such as tetrahydrofuran, diethyl ether, diisopropylether, dioxane, dimethoxyethane and the like; halogenated hydrocarbons such as chloroform, carbon tetrachloride, 1,2-dichloroethane and the like; hydrocarbons such as hexane, haptane and the like; aromatic hydrocarbons such as benzene, toluene and the like; and mixtures thereof, preferably tetrahydrofuran.

Means for removal of remaining palladium resulting from a palladium catalyst to be used in this step may include, but are not limited to, use of sulfur-containing compounds such as trimercaptotriazine, sodium dimethyldithiocarbamate and the like; use of resin fixing type adsorbing agents such as DiaIon CR20 (registered trademark) and the like; use of column chromatography such as silica gel column and the like. Among them, use of sulfur-containing compounds such as trimercaptotriazine, sodium dimethyldithiocarbamate and the like are preferable.

The eighth step of the present preparation method is a process in which sodium ions are added to a compound of the formula (II) to prepare a compound of the formula (I).

The sodium source for a sodium ion to be used in this step is not particularly limited, and may include sodium hydroxide, sodium carbonate and the like. Among them, sodium hydroxide is preferable.

The solvent to be used in this step is not particularly limited. It is desirable that the solvent may be one of inert solvents not reacting easily with a raw material. Examples thereof may include alcohols such as methanol, ethanol, isopropanol, tert-butanol and the like; ethers such as tetrahydrofuran, diethyl ether, diisopropyl ether, dioxane, dimethoxyethane and the like; acetate esters such as ethyl acetate, methyl acetate, isopropyl acetate and the like; ketones such as acetone, methyl ethyl ketone and the like; nitriles such as acetonitrile and the like; water; and mixed solvents thereof; and the like. Among them, alcohols such as methanol, ethanol, isopropanol, tert-butanol and the like are preferable.

The compound of the formula (I) of the present invention is particularly useful as a preventive or therapeutic agent for sepsis, endotoxemia and prognosis of coronary-artery bypass graft surgeries (CABG) since it antagonizes lipid A playing an important role in Gram negative bacteremia, in particular endotoxin shock, manifesting high fatality rate caused by lipopolysaccharide (LPS) components or endotoxin present in Gram negative outer membrane, shows an excellent anti-endotoxin action, and shows an antagonistic action on TLR4 (toll-like receptor 4) which is one of receptors recognizing a fungus body component of a bacterium.

### Examples:

The present invention will be further illustrated by way of the following examples, but is not limited thereto.

Identification of the compound according to the present invention was carried out by using a compound synthesized according to the preparation method described in WO 2004/074303 (Patent Document 3) as a control and by comparing the retention time by a HPLC method. Quantification of the compound was calculated from strength obtained by a UV detector by HPLC method, from a calibration curve based on a compound synthesized according to the preparation method described in WO 2004/074303 (Patent Document 3) as a control.

The stationary phase which can be used in a HPLC method is not particularly limited, and reverse phase columns such as C18 (ODS), C4, C8, C22, C30 and the like are preferable. The mobile phase is not particularly limited, and solvents such as acetonitrile, methanol, water and the like or mixed solvents thereof are preferable. If desired, excellent peak separation is obtained by adding acids such as perchloric acid, trifluoroacetic acid, acetic acid, phosphoric acid and the like and salts thereof, or amines such as triethylamine, diethylamine and the like. Reproducibility of peak separation and retention time is improved by keeping the column temperature at constant level by a column oven and the like.

### Example 1:

### α-D-glucopyranose, (1Z)-1-propenyl 2-deoxy-3-O-[(3R)-3-methoxydecyl]-6-O-methyl-2-[[(11Z)-1-ox o-11-octadecenyl]amino]-, 4-(di-2-propenyl phosphate)

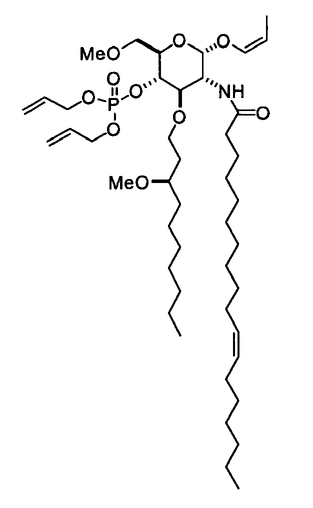

In a 2 L four-necked flask, 235 g of α-D-glucose, (1Z)-1-propenyl 2-deoxy-3-O-[(3R)-3-methoxydecyl]-6-O-methyl-2-[[(11Z)-1-ox O-11-octadecenyl]amino]- [CAS registered number: 748165-17-5] was dissolved in 933 mL of toluene. Then, to the mixture, 129 mL of diallyl N,N-diisopropylphosphoramidate, 39.4 mL of pyridine and 36.3 mL of trifluoroacetic acid were added dropwise sequentially at room temperature. After 1. 5 hours of completion of adding, the reaction solution was cooled down to -20°C, and an acetonitrile diluted solution (933 mL) containing 47.5 mL of hydrogen peroxide was added dropwise over 37 minutes. After completion of adding, the temperature was raised up to 10°C over a period of 40 minutes. After 3 hours, 940 mL of a 5% sodium hydrogen sulfite aqueous solution was added to quench the reaction, and the temperature was raised up to room temperature. After extracting with ethyl acetate, the solution was cold-stored and used as it was in the subsequent reaction as a solution of the titled compound.

### Example 2:

### α-D-glucose, 2-deoxy-3-O-[(3R)-3-methoxydecyl]-6-O-methyl-2-[[(11Z)-1-ox O-11-octadecenyl]amino]-, 4-(di-2-propenyl phosphate)

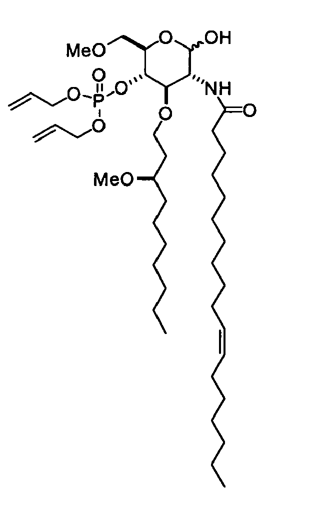

The solution of α-D-glucopyranose, (1Z)-1-propenyl 2-deoxy-3-O-[(3R)-3-methoxydecyl]-6-O-methyl-2-[[(11Z)-1-ox O-11-octadecenyl]amino]-, 4-(di-2-propenyl phosphate), obtained in Example 1 , was washed with 699 mL of 1 N hydrochloric acid. To the resultant, 27.9 mL of 5 N hydrochloric acid was added, and the solution was stirred at room temperature for 5 hours. The solution was neutralized with 699 mL of 5% sodium bicarbonate aqueous solution, then, separated with ethyl acetate. An organic layer was washed with 699 mL of 5% saline. To the resultant, 69.9 g of anhydrous magnesium sulfate was added for drying and filtrated. The filtrate was concentrated under reduced pressure. To the residue was added 466 mL of acetone, and concentrated again under reduced pressure. The acetone treatment was repeated to obtain 289.1 g of a crude material of the titled compound (content ratio: 92. 1%, content: 266.3%). Yield: 97%.

To 289.1 g of the resulting crude material was added 1065 mL of acetonitrile and the mixture was stirred at 20°C for 5 minutes. Then, the mixture was cooled down to 0°C over 4 hours, and further stirred for 4 hours. The deposited crystal was filtrated, and dried over night under reduced pressure at room temperature, to obtain the titled compound of an amount corresponding to 228.6 g.

### Example 3:

### α-D-glucopyranose, 2-deoxy-3-O-[(3R)-3-methoxydecyl]-6-O-methyl-2-[[(11Z)-1-ox o-11-octadecenyl]amino]-, 4-(di-2-propenyl phosphate) 1-(2,2,2-trichloroethaneimidate)

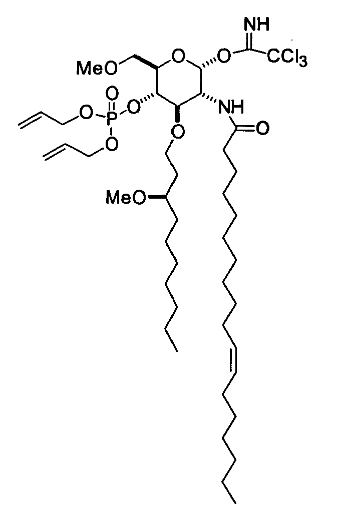

Into a 2 L four-necked flask was added 280 g of α-D-glucose, 2-deoxy-3-O-[(3R)-3-methoxydecyl]-6-O-methyl-2-[[(11Z)-1-ox o-11-octadecenyl]amino]-, 4-(di-2-propenyl phosphate), 46. 8 g of potassium carbonate, 560 mL of methyl acetate, 170 mL of trichloroacetonitrile and 8.4 mL of water. The mixture was stirred for 2 hours at 0°C under a nitrogen atmosphere. The reaction solution was filtrated through celite and concentrated at 40°C under reduced pressure. Subsequently, azeotropy was performed 3 times with 560 mL of heptane, to obtain 432 g of the titled compound (content ratio: 63.9%, containing 171.4 mL of heptane). Yield: 87.5%.

### Example 4:

### α-D-glucopyranoside, (1Z)-1-propenyl 6-O-[4-O-[bis(2-propenyloxy)phosphinyl]-2-deoxy-3-O-[(3R)-3 -methoxydecyl]-6-O-methyl-2-[[(11Z)-1-oxo-11-octadecenyl]am ino]-β-D-glucopyranosyl]-3-O-decyl-2-deoxy-2-[(1,3-dioxotet radecyl)amino]-, 4- (2-propenyl carbonate)

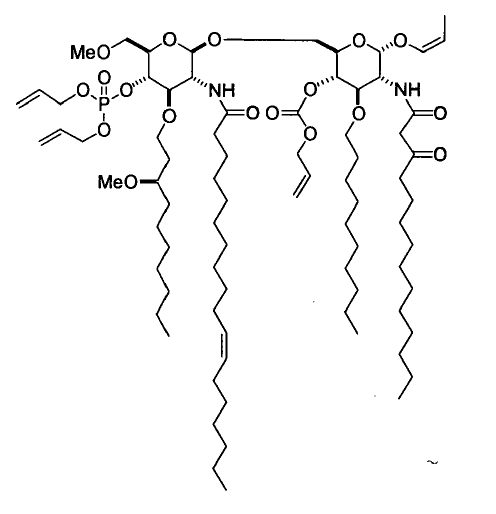

Into a 2 L four-necked flask was added sequentially a heptane solution (content ratio: 50.4%) of 410.8 g of α-D-glucopyranose, 2-deoxy-3-O-[(3R)-3-methoxydecyl]-6-O-methyl-2-[[(11Z)-1-ox O-11-octadecenyl]amino]-, 4-(di-2-propenyl phosphate) 1-(2,2,2-trichloroethaneimidate), 249.7 mL of heptane, 105.9 g of α-D-glucopyranoside, (1Z)-1-propenyl 3-O-decyl-2-deoxy-2-[(1,3-dioxotetradecyl)amino]-, 4-(2-propenyl carbonate) [CAS registered number: 185955-29-7], 140 mL of toluene and 2.89 mL of methanesulfonic acid. The mixture was stirred for 15 hours at 25°C under a nitrogen atmosphere. To the reaction solution were added 2000 mL of ethyl acetate and 1000 mL of water. The solution was separated, and then, an organic layer was washed sequentially with 1000 mL of a 5% sodium hydrogen carbonate aqueous solution and 1000 mL of 10% saline. The solution was concentrated under reduced pressure (warm bath: 45 to 50°C), then, 800 mL of methanol was added to the residue and the mixture was concentrated, further, the same operation was repeated to obtain a crude material of the titled compound.

To the resultant crude material was added 1920 mL of methanol, and insoluble substances were filtrated through celite. The insoluble substances and celite were washed with methanol. Further, 1400 mL of methanol was added to the solution, then, the mixture was cooled to 17°C and 375 mL of water was added dropwise. Thereafter, the mixture was cooled down to -20°C and the mixture was stirred for 45 minutes, then, filtrated. The filtrate was washed with 400 mL of 90% water-containing methanol cooled previously down to 0°C, and the mixture was itself dried under reduced pressure on Buchner funnel, to obtain 427.2 g of a wet substance.

Into a 10 L four-necked flask was added 427.2 g of the wet substance, and 2400 mL of methanol was added to dissolve the wet substance. The solution was cooled to 10°C, and then, 180 mL of water was added dropwise. After completion of adding, the solution was cooled to 0°C, and stirred for 50 minutes, then, filtrated. The filtrate was washed with 400 mL of 90% water-containing methanol cooled previously down to 0°C, then, dried under reduced pressure at 35°C, to obtain 199.5 g of the titled compound (content ratio: 92.2%). Yield: 92.6%.

### Example 5:

### α-D-glucopyranose, 6-O-[4-O-[bis(2-propenyloxy)phosphinyl]-2-deoxy-3-O-[(3R)-3-methoxydecyl]-6-O-methyl-2-[[(11Z)-1-oxo-11-octadecenyl]amino]-β-D-glucopyranosyl]-3-O-decyl-2-deoxy-2-[(1,3-dioxotetradecyl)amino]-, 4-(2-propenyl carbonate)

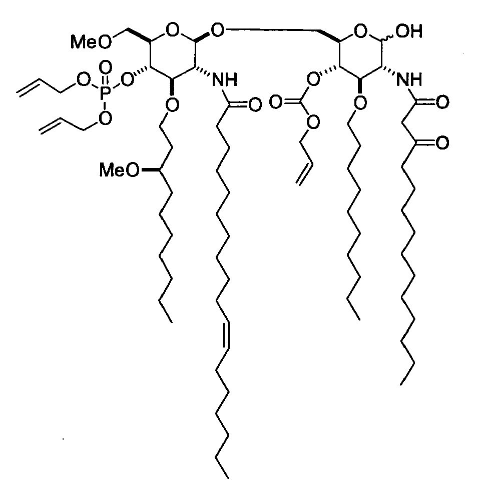

Into a 10 L four-necked flask was added 199.0 g (content ratio: 92.2%) of α-D-glucopyranoside, (1Z)-1-propenyl 6-O-[4-O-[bis(2-propenyloxy)phosphinyl]-2-deoxy-3-O-[(3R)-3 -methoxydecyl]-6-O-methyl-2-[[(11Z)-1-oxo-11-octadecenyl]am ino]-β-D-glucopyranosyl]-3-O-decyl-2-deoxy-2-[(1,3-dioxotet radecyl)amino]-, 4-(2-propenyl carbonate), 1990 mL of acetonitrile and 34.6 mL of 1 N hydrochloric acid. The mixture was stirred at 30°C for 2 hours under 130 hPa. Further, the pressure reduction and jacket temperature were raised gradually, and finally, acetonitrile was concentrated to a volume of about 3/4 under 106 hPa. To the concentrated solution was added 995 mL of 10% saline and 1493 mL of ethyl acetate, followed by extraction. Thereafter, an organic layer was washed sequentially with 995 mL of 5% sodium hydrogen carbonate water and 995 mL of 10% saline. An organic layer was dried over 60 g of anhydrous magnesium sulfate, then, filtrated. The filtrate was concentrated. To the residue was added 640 mL of toluene to dissolve the residue, obtaining 778.1 g (content ratio: corresponding to 155.6 g) of a toluene solution of the titled compound. Yield: 87.2%.

### Example 6:

### α-D-glucopyranose, 6-O-[4-O-[bis(2-propenyloxy)phosphinyl]-2-deoxy-3-O-[(3R)-3-methoxydecyl]-6-O-methyl-2-[[(11Z)-1-oxo-11-octadecenyl]amino]-β-D-glucopyranosyl]-3-O-decyl-2-deoxy-2-[(1,3-dioxotetradecyl)amino]-, 1-(di-2-propenyl phosphate) 4-(2-propenylcarbonate)

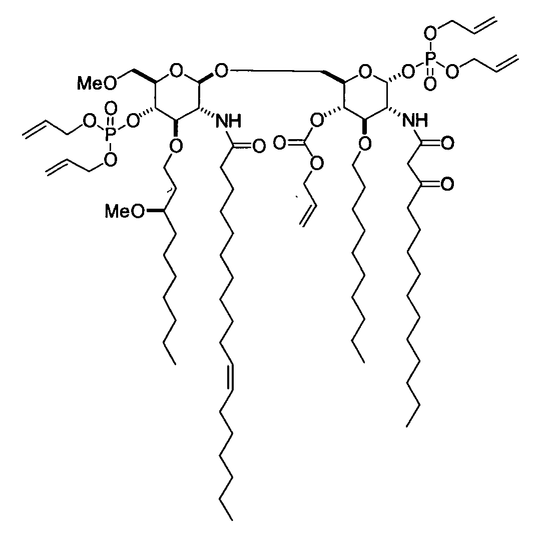

550.6 g (content ratio: corresponding to 110 g) of a toluene solution of α-D-glucopyranose, 6-O-[4-O-[bis(2-propenyloxy)phosphinyl]-2-deoxy-3-O-[(3R)-3-methoxydecyl]-6-O-methyl-2-[[(11Z)-1-oxo-11-octadecenyl]amino]-β-D-glucopyranosyl]-3-O-decyl-2-deoxy-2-[(1,3-dioxotetradecyl)amino]-, 4-(2-propenyl carbonate) was concentrated under reduced pressure at 50°C. To the residue was added 440 mL of toluene to dissolve the residue, and the solution was concentrated under reduced pressure at a bath temperature of 45 to 50°C. Further, 440 mL of toluene was added, then, an atmosphere was replaced with nitrogen to obtain 537.6 g (content: 109.13g) of a toluene solution. This solution was concentrated under reduced pressure, then, 665 mL of anhydrous toluene was added, and an atmosphere was replaced with nitrogen. 11.91 mL of trifluoroacetic acid was added and the mixture was stirred for 15 hours, then, 12. 50 mL of pyridine was added. The mixture was cooled to -20°C, then, 37.15 mL of diallyl N,N-diisopropylphosphoramidate was added dropwise. After 30 minutes of completion of adding, the mixture was cooled down to -30°C, and 15.17 mL of 30% hydrogen peroxide was added dropwise. After 6 minutes of completion of adding, a thermostat was set at -20°C. One hour and 10 minutes after, 655 mL of 5% sodium thiosulfate aqueous solution was added to quench the reaction. 655 mL of ethyl acetate was added and extracted. An organic layer was washed sequentially with 655 mL of 0.5 N hydrochloric acid, 655 mL of 10% saline, 655 mL of 5% sodium bicarbonate aqueous solution and 655 mL of 10% saline. 43. 7 g of anhydrous magnesium sulfate was added for drying, then, the product was filtrated. The filtrate was concentrated under reduced pressure to obtain 159.0g of the titled compound (content: 101.6g). Yield: 83.5%.

### Example 7:

### α-D-glucopyranose, 3-O-decyl-2-deoxy-6-O-(2-deoxy-3-O-[(3R)-3-methoxydecyl]-6-O-methyl-2-[[(11Z)-1-oxo-11-octadecenyl]amino]-4-O-phosphon O-β-D-glucopyranosyl)-2-[(1,3-dioxotetradecyl)amino]-, 1-(dihydrogen phosphate), 4 sodium salt

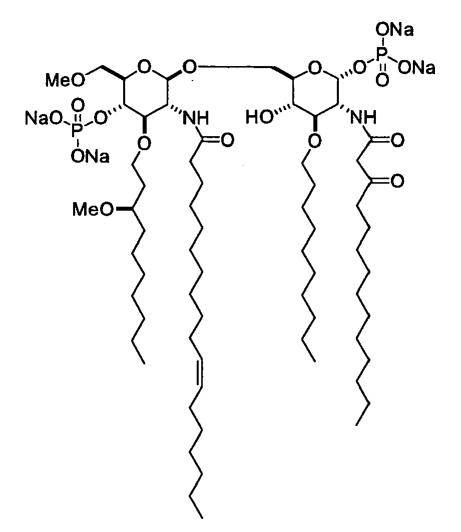

Into a 3 L four-necked flask was charged 70.49 g of Meldrum's acid, 2.93 g of palladium acetate and 51.3 g of triphenylphosphine. After an atomosphere was replaced with nitrogen, 1321 mL of tetrahydrofuran was added, and a tetrahydrofuran solution (203 mL) of 101.6 g of α-D-glucopyranose, 6-O-[4-O-[bis(2-propenyloxy)phosphinyl]-2-deoxy-3-O-[(3R)-3 -methoxydecyl]-6-O-methyl-2-[[(11Z)-1-oxo-11-octadecenyl]am ino]-β-D-glucopyranosyl)-3-O-decyl-2-deoxy-2-[(1,3-dioxotetradecyl)amino]-, 1-(di-2-propenyl phosphate) 4-(2-propenyl carbonate) was added. The mixture was stirred at 32°C for 2 hours, then, further stirred at 30°C for 4 hours. To the reaction solution was added 250 mL of methanol, and the mixture was concentrated under reduced pressure, to obtain 466.7 g of a residue. To the residue was added 4570 mL of methanol and heated up to 40°C to dissolve the residue. Then, 5.55 g of trimercaptotriazine was added, and the mixture was stirred overnight at room temperature. The deposited trimercaptotriazine-palladium complex was filtrated, further, washed with methanol, to obtain 4330 g of a filtrate.

3908.2mL of this methanol solution was concentrated under reduced pressure, to obtain 440. 9 g of a residue. To the residue was added 450 mL of acetone, the mixture was concentrated under reduced pressure. Then, 450 mL of acetone was again added and the solution was concentrated. The residue was cold-stored overnight, then, 1800 mL of acetone was added, and the mixture was heated to 40°C, and stirred for 1.5 hours. The resulting mixture was air-cooled and stirred for 1.5 hours at 30°C or lower, then, filtrated. The filtrate was washed with 750 mL of acetone, and the collected solid was dried under reduced pressure at 35 to 40°C, to quantitatively obtain 104. 48 g (content ratio: 74.2%) of a free form of the titled compound as a crude material.

The resulting crude material was treated with 0.1 N sodium hydroxide aqueous solution, to obtain the titled compound.

## Claims

1. A method for preparing a compound represented by following formula (I), comprising the steps of:
reacting a compound represented by following formula (VIII) with a palladium catalyst in the presence of a nucleophilic reagent; and
then treating the resultant with a sodium source to obtain the compound represented by the formula (I):

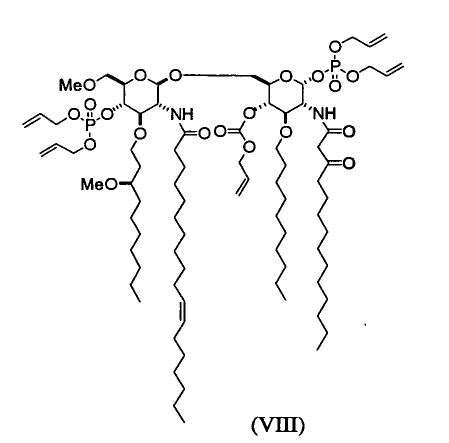

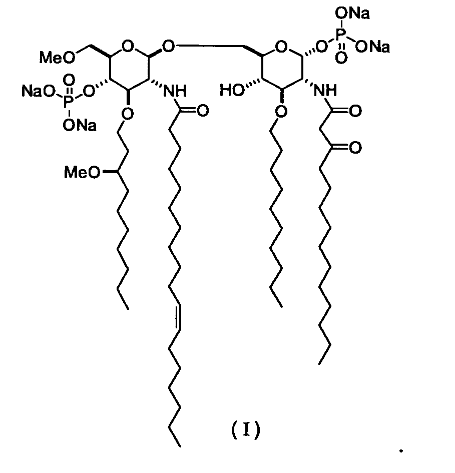

2. A method for preparing a compound represented by following formula (I), comprising the steps of:
reacting a compound represented by following formula (VII), diallyl N,N-diisopropylphosphoramidate and an oxidizing agent in this order, in a first aromatic hydrocarbon solvent in the presence of pyridine-trifluoroacetic acid, to obtain a compound represented by following formula (VIII);
reacting the compound represented by the formula (VIII) with a palladium catalyst in the presence of a nucleophilic reagent; and
then treating the resultant with a sodium source to obtain the compound represented by the formula (I):

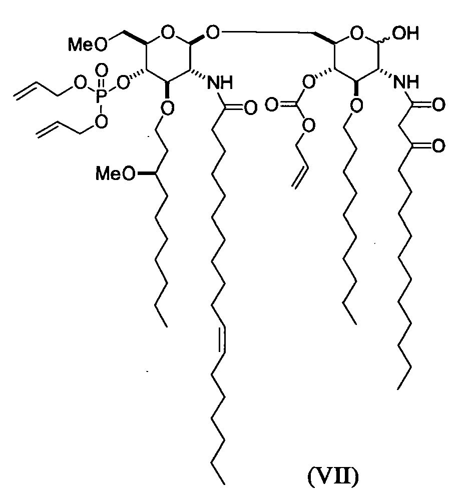

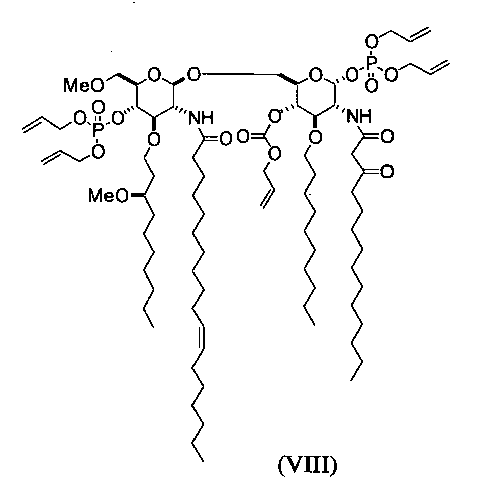

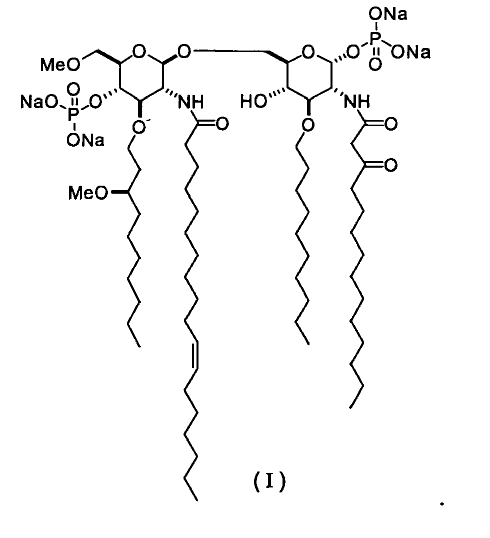

3. A method for preparing a compound represented by following formula (I), comprising the steps of:
selectively deprotecting a 1-propenyl group of a compound represented by following formula (VI), to obtain a compound represented by following formula (VII);
reacting the compound represented by the formula (VII), diallyl N,N-diisopropylphosphoramidate and an oxidizing agent in this order, in a first aromatic hydrocarbon solvent in the presence of pyridine-trifluoroacetic acid, to obtain a compound represented by following formula (VIII);
reacting the compound represented by the formula (VIII) with a palladium catalyst in the presence of a nucleophilic reagent; and
then treating the resultant with a sodium source to obtain the compound represented by following formula (I):

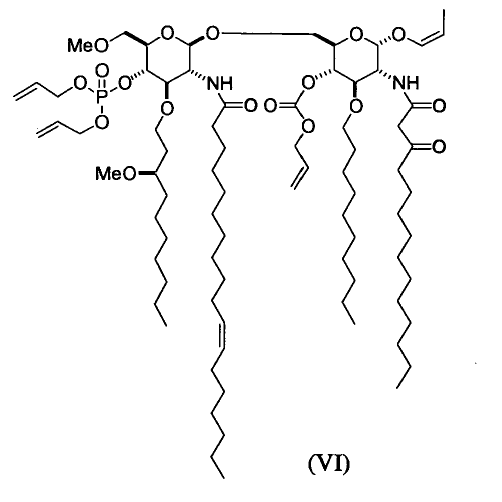

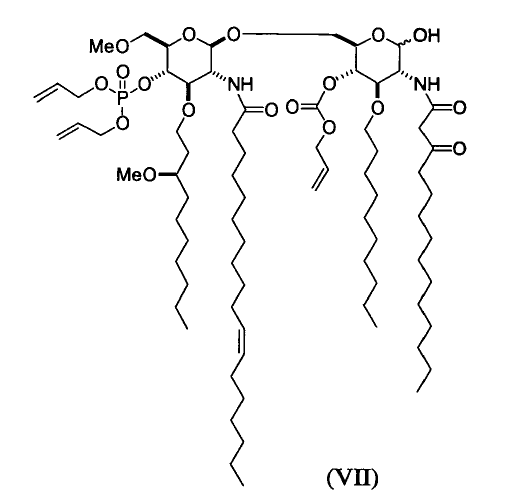

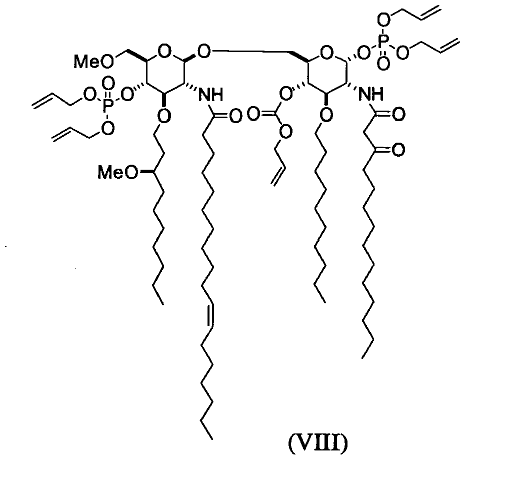

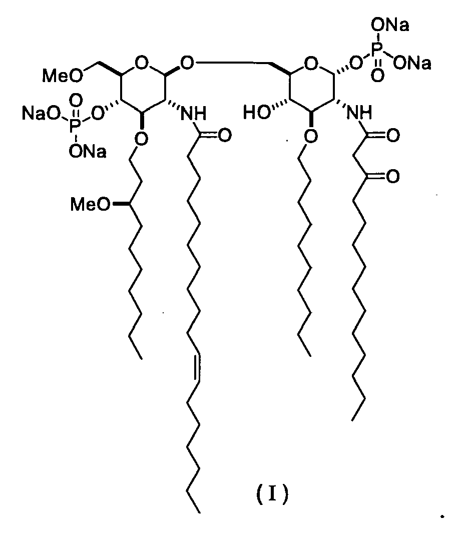

4. A method for preparing a compound represented by following formula (I), comprising the steps of:
reacting a compound represented by following formula (IV) and a compound represented by following formula (V) in a first solvent comprising a hydrocarbon solvent and/or a second aromatic hydrocarbon solvent, in the presence of organic sulfonic acid, to obtain a compound represented by following formula (VI);
selectively deprotecting a 1-propenyl group of the compound represented by the formula (VI), to obtain a compound represented by following formula (VII);
reacting the compound represented by the formula (VII), diallyl N,N-diisopropylphosphoramidate and an oxidizing agent in this order, in a first aromatic hydrocarbon solvent in the presence of pyridine-trifluoroacetic acid, to obtain a compound represented by following formula (VIII);
reacting the compound represented by the formula (VIII) with a palladium catalyst in the presence of a nucleophilic reagent; and
then treating the resultant with a sodium source to obtain the compound represented by the formula (I):

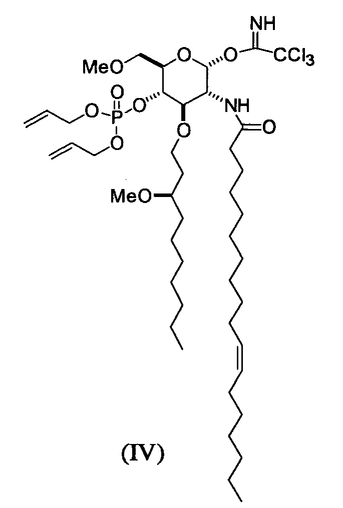

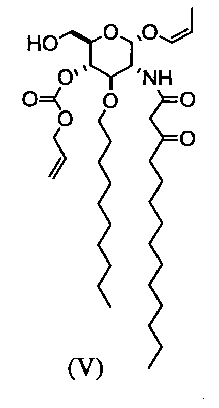

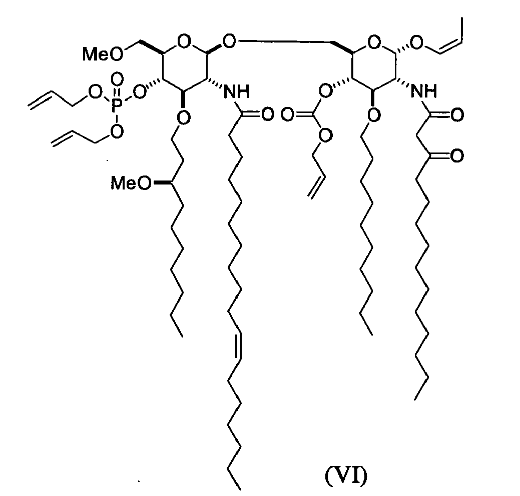

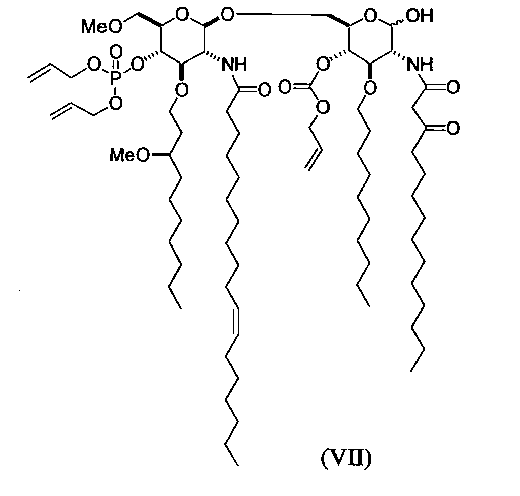

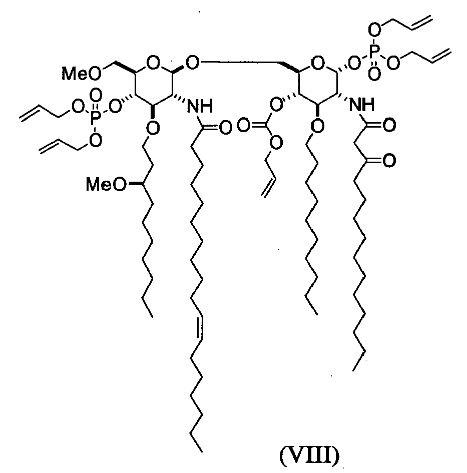

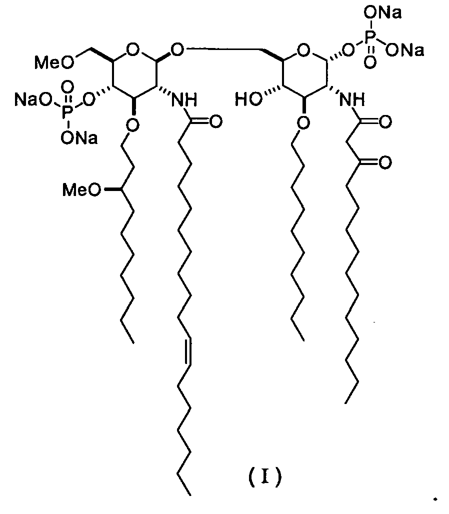

5. The method according to any one of claims 1 to 4, wherein the first aromatic hydrocarbon solvent is a toluene solvent.

6. The method according to claim 4 or 5, wherein the organic sulfonic acid is methanesulfonic acid or ethanesulfonic acid.

7. The method according to any one of claims 4 to 6, wherein the first solvent is a toluene-heptane mixed solvent.

8. The method according to any one of claims 4 to 7, wherein the compound represented by the formula (IV) is obtained by reacting a compound represented by following formula (III) and trichloroacetonitrile in a mixed solvent of an acetate ester solvent and water, in the presence of potassium carbonate, in which an amount of trichloroacetonitrile ranges 1 to 10 equivalents based on 1 equivalent of the compound represented by the formula (III):

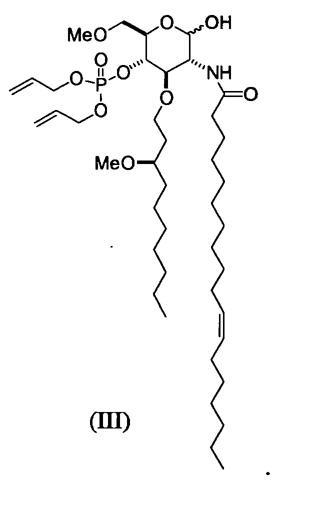

9. The method according to any one of claims 4 to 8, wherein the compound represented by the formula (IV) is obtained by selectively deprotecting a 1-propenyl group of a compound represented by following formula (X), to obtain a compound represented by following formula (III), and
then reacting the compound represented by the formula (III) and trichloroacetonitrile in a mixed solvent of an acetate ester solvent and water, in the presence of potassium carbonate, in which an amount of trichloroacetonitrile ranges 1 to 10 equivalents based on 1 equivalent of the compound represented by the formula (III):

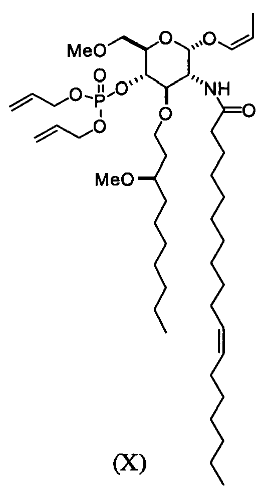

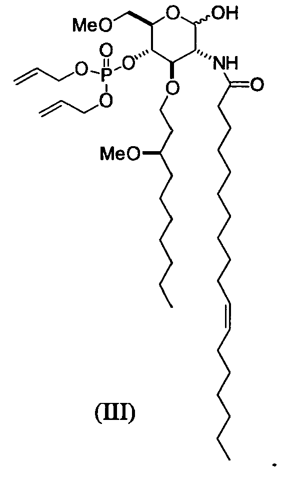

10. The method according to any one of claims 4 to 9, wherein the compound represented by the formula (IV) is obtained by reacting a compound represented by following formula (IX) with diallyl N,N-diisopropylphosphoramidate and an oxidizing agent in this order, in the presence of pyridine-trifluoroacetic acid, to obtain a compound represented by following formula (X);
selectively deprotecting a 1-propenyl group of the compound represented by the formula (X), to obtain a compound represented by following formula (III), and
then reacting the compound represented by the formula (III) and trichloroacetonitrile in a mixed solvent of an acetate ester solvent and water, in the presence of potassium carbonate, in which an amount of trichloroacetonitrile ranges 1 to 10 equivalents based on 1 equivalent of the compound represented by the formula (III):

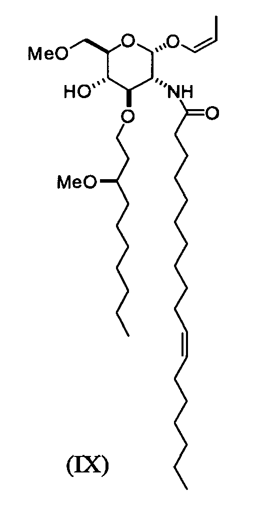

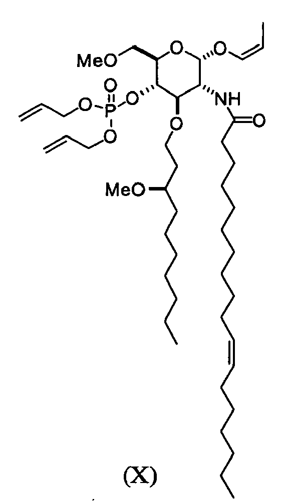

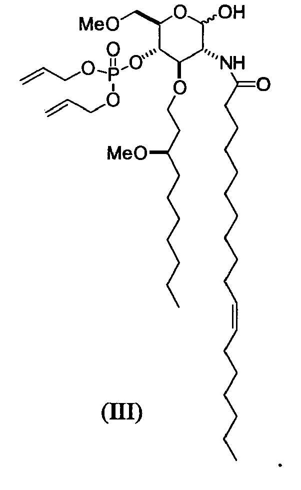

11. The method according to any one of claims 8 to 10, wherein the acetate ester solvent is methyl acetate.

12. The method according to any one of claims 8 to 11, wherein an amount of the water in the mixed solvent ranges 1 to 10 percent (vol/vol ratio).

13. The method according to any one of claims 1 to 12, wherein the nucleophilic reagent is cyclic organic acid esters or cyclic ketones.

14. The method according to any one of claims 1 to 13, wherein the nucleophilic reagent is Meldrum's acid or Dimedone.

15. The method according to any one of claims 1 to 14, wherein the palladium catalyst is tetrakis(triphenylphosphine) palladium.

16. The method according to claim 15, wherein the tetrakis (triphenylphosphine) palladium is prepared in situ from palladium acetate and triphenylphosphine.
